(19) Europäisches Patentamt
European Patent Office
Office européen des brevets

(11) EP 4 397 651 A1

(12) EUROPEAN PATENT APPLICATION

(43) Date of publication:
**10.07.2024 Bulletin 2024/28**

(21) Application number: **23150108.1**

(22) Date of filing: **03.01.2023**

(51) International Patent Classification (IPC):
***C07C 69/82*** *(2006.01)* ***C08J 11/14*** *(2006.01)*
***C08J 11/24*** *(2006.01)* ***C08J 11/28*** *(2006.01)*

(52) Cooperative Patent Classification (CPC):
(C-Sets available)
**C08J 11/28; C07C 67/03; C08J 11/14; C08J 11/16; C08J 11/24;** C08J 2367/02 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Københavns Universitet**
**1165 Copenhagen K (DK)**

(72) Inventors:
- **Lee, Jiwoong**
  **1165 Copenhagen K (DK)**
- **Yang, Yang**
  **1165 Copenhagen K (DK)**
- **Sharma, Shriaya**
  **1165 Copenhagen K (DK)**
- **Kamounah, Fadhil S.**
  **1165 Copenhagen K (DK)**
- **di Bernado, Carlo**
  **1165 Copenhagen K (DK)**

(74) Representative: **Plougmann Vingtoft a/s**
**Strandvejen 70**
**2900 Hellerup (DK)**

# (54) CATALYSTS FOR POLYETHYLENE TEREPHTHALATE DEGRADATION

(57) The present invention has been made within the field of plastic/polymer recycling and relates to a process for transforming e.g. end-of-use/waste PET polymer (a very common polyester) into a commonly used and pure monomer compound (e.g. BHET), which can re-enter the production of "pristine" PET, i.e. PET with similar or the same quality as non-recycled PET. Furthermore, the present invention can be used to recover both BHET and textile e.g. cotton from PET/textile blended fabric.

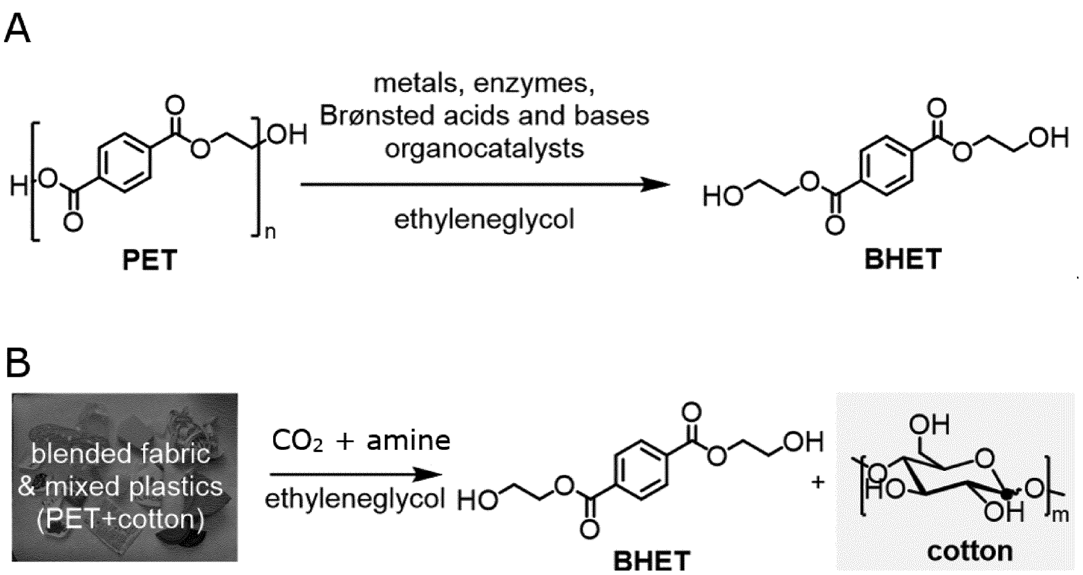


Fig. 1

EP 4 397 651 A1

(52) Cooperative Patent Classification (CPC): (Cont.)

C-Sets
**C07C 67/03, C07C 69/82**

## Description

### Technical field of the invention

The present invention has been made within the field of chemistry, more specifically plastic/polymer recycling and relates to a process for transforming e.g. end-of-use polyethylene terephthalate (PET) polymer into a commonly used monomer compound such as bis(hydroxyethyl)terephthalate (BHET), which can re-enter the production of pristine PET, i.e. PET with similar or equal quality as non-recycled PET. Furthermore, the present invention can be used to recover both BHET and textile, such as cotton, from blended fabrics.

### Background of the invention

Synthetic polymers are amongst the greatest polluting concerns preventing sustainable growth of human society. Worldwide polymer production (ca. 348 million metric tons world production in 2017) has grown exponentially throughout the years. Polyethylene terephthalate (PET) is one of the most produced polymers - approximately 70 million metric tons per year and it is expected to grow more than 4% annually. PET-based polymers are mainly used in beverage bottles, while a third of the produced PET is blended with cotton-based fibers and viscose to produce fabrics for the textile industry. Consequently, recycling of these composite materials including beverage bottle wastes becomes an urgent societal issue. Chemical upcycling - also referred to as chemical recycling, monomer production and chemical decomposition - of plastic wastes has been an attractive option to produce higher-value products while avoiding additional greenhouse gas emission and negative environmental impact from energy recovery in waste incinerators.

Catalytic glycolysis and transesterification of PET bottles is an atom-economic approach affording the useful intermediate BHET (bis(hydroxyethyl)-terephthalate), which can be recycled as a precursor for producing PET via polymerization. Lewis acidic metals have been employed commercially in these reactions as a catalyst at high reaction temperatures (160 - 220 °C) to overcome the high reaction barrier and to accelerate solubilization of PET (Figure 1A). Metal-catalyzed PET glycolysis can be exemplified with the catalysts $Zn(OAc)_2$, Ti(IV) phosphate, and $Mn(OAc)_2$ which show satisfactory performance (see for example: M.Ghaemy et al., Polymer Degradation and Stability, Volume 90, Issue 3, December 2005, Pages 570-576). Although these catalytic systems exhibit high efficiency, only a few protocols can be implemented in generating high purity BHET due to the metal contamination, thus, requiring tedious purification steps of crystallization of BHET, reducing compatibility of produced BHET in repolymerization to PET.

Contamination with heavy metals is a significant issue in polymerization of BHET obtained from the above methods in terms of yield and appearance of the PET polymers. Metal contamination of e.g. cotton in depolymerization of PET in mixed PET-cotton fabrics is also an issue for these methods. Organocatalysis may present a solution to such problems, and has been studied in this regard.

For example, WO 2015/056377 discloses a process for converting PET to BHET using various organic amine bases, such as for example trimethylamine (TEA), ethylene glycol at 220 °C and 20 psi pressure (Example 3, page 21). No source of $CO_2$ is applied. High ratios of BHET to PET oligomers are reported in the depolymerization product. This method however has the disadvantage of using more toxic and costly amine catalysts, and harsher conditions including pressure and/or temperature) to obtain the yields reported.

Hence, an improved method of converting PET back to its monomer component BHET or useful derivatives thereof would be advantageous, and in particular a more high-yielding, environmentally friendly and facile method for producing BHET monomers from PET, and for separating PET from other textiles in blended fabrics would be advantageous.

### Summary of the invention

Thus, an object of the present invention is to provide a method for producing BHET monomer or derivatives thereof from end-of-use/waste PET material, with the aim of providing a monomer that may produce recycled PET of similar quality as non-recycled PET.

In particular, it is an object of the present invention to provide a method that solves the above mentioned problems of the prior art with metal contaminants and undesired pigmentation of BHET from depolymerized PET and the resulting recycled PET material, while avoiding the use of toxic bases and high pressure and/or temperature.

Thus, a first aspect of the present invention is a process for chemical depolymerisation of poly(ethylene)terephthalate (PET) comprising the steps of:

a) providing a material comprising PET;

b) mixing the material comprising PET from step a) with a source of carbon dioxide, a source of amine, and a compound of formula (I):

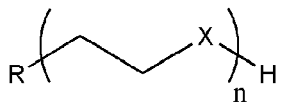 (I);

c) heating the mixture obtained in step b) to a temperature in the range of 130°C to 220°C;
d) obtaining a product comprising a compound of formula (II):

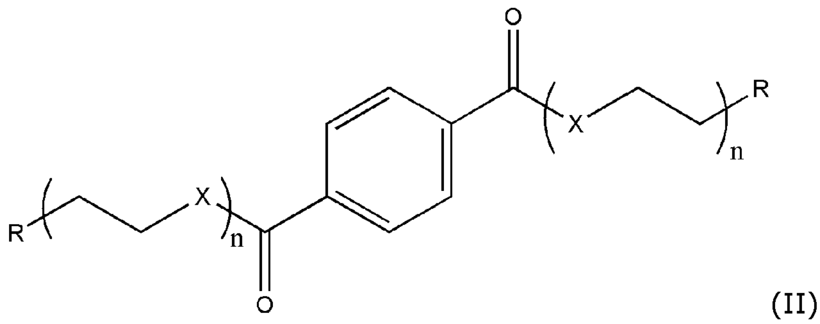

(II);

wherein

R is selected from the group consisting of OH and $N(R^1)_2$,
X is selected from the group consisting of O and $NR^2$,
$R^1$ and $R^2$ are independently selected from the group consisting of H, methyl, ethyl, propyl, isopropyl, butyl, and isobutyl,
n is any integer in the range of 1-10.

Another aspect of the present invention is a process for separating poly(ethylene)terephthalate (PET) and a textile from a blended fabric comprising PET and textile, said process comprising the steps of:

a) providing a blended fabric comprising textile and PET;
b) mixing the blended fabric from step a) with a source of carbon dioxide, a source of amine, and a compound of formula (I):

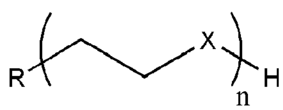 (I);

c) heating said mixture obtained in step b) to a temperature in the range of 130°C to 220°C;
d) obtaining a product comprising a compound of formula (II):

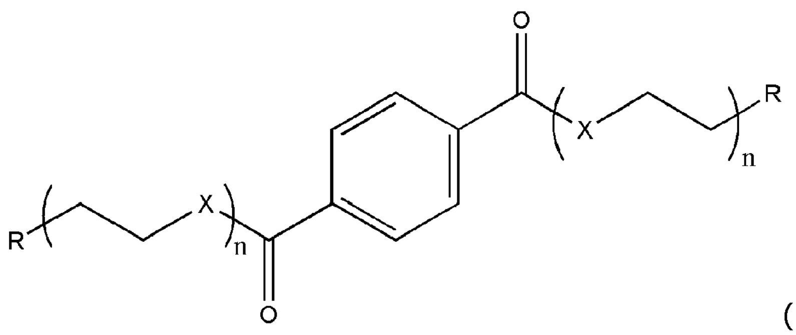

(II)

and textile;
e) separating the compound of formula (II) and textile of said product of step d);

wherein

R is selected from the group consisting of OH and $N(R^1)_2$,
X is selected from the group consisting of O and $NR^2$,
$R^1$ and $R^2$ are independently selected from the group consisting of H, methyl, ethyl, propyl, isopropyl, butyl, and isobutyl,
n is any integer in the range of 1-10.

The present inventors have surprisingly found that these processes provides for BHET derivatives in high yields from the starting PET material, and is tolerant to the presence of many other materials in the reaction mixture, while allowing for easy separation of the product from the catalyst and solvents used.

**Brief description of the figures**

**Figure 1** shows **(A)** an overview depiction of prior art chemical recycling of PET via glycolysis using metal catalysts, and **(B)** the COz and $NH_3$-catalyzed chemical recycling of drinking bottles and textile wastes used in the present invention.

**Figure 2A** shows $NH_4HCO_3$-catalytic transesterification of ethyl esters **1** to ethylene glycol esters **3** using ethylene glycol **2a,** as compared to the same reaction with no catalyst. Yields in black are the $NH_4HCO_3$-catalytic transesterification, while yields in parentheses are without catalyst. [b]Methyl benzoate was used as a starting material. [c]Reactions performed at 150 °C with 25 mol% catalyst. $NH_4HCO_3$ Catalysed yields are shown to be significantly better than with no catalyst.

**Figure 2B** shows $NH_4HCO_3$-catalytic transesterification of ethyl esters **1a** to esters **3a-k** using ethylene glycol and PEG derivatives **2a-k.** NMR yields of the product is shown in percentages unless otherwise indicated. The results illustrates the substrate flexibility in terms of the compound of formula (I).

**Figure 3A** shows an exemplary reaction scheme for the catalytic depolymerisation of PET and recovery of cotton.

**Figure 3B** shows the reaction progress for ethylene glycolysis of PET polyester (75%) in the presence of cotton with* and without" catalyst ($NH_4HCO_3$) at 180 °C and 190 ° respectively. The catalysed reaction proceeds very well even at 180 °C-190 °C, while the un-catalysed reaction provides below 50% yield after 6 h.

**Figure 4A** shows chemical recycling of PET/textile blended fabrics on a large scale from garments and furniture material. Glycolysis is performed with fabrics having 47-100% PET, showing optical images and yield percentages of recovered cotton/textile materials and BHET. Reactions show excellent recovery of textile and good yields of BHET.

**Figure 4B** shows Solid-state $^{13}C$ NMR analysis of the recovered cotton from a reaction with $NH_4HCO_3$ (fourth panel from the top), without $NH_4HCO_3$ (third panel from the top), commercial cotton ball (second panel from the top) and the starting material (first panel from the top). It is seen that the spectrum for recovered cotton is similar to that of commercial cotton balls.

**Figure 4C** shows optical microscope images of recovered cotton from reaction with $NH_4HCO_3$ (right panel) and recovered cotton from reaction with $Zn^{2+}$ (left panel) at at 20 times, 40 times and 80 times magnification. The superior quality of cotton recovered from the method of the present invention is clearly visible.

**Figure 4D** shows optical microscope images of a commercial cotton ball (top, left panel), the starting material (top, right panel), recovered cotton from reaction without (bottom, left panel) and with $NH_4HCO_3$ (bottom, right panel). Improved removal of PET fibres is visible for the reaction of the present invention.

**Figure 5A** shows thermal-gravimetric analysis (TGA) results from chemical recycling of cotton from PET/cotton blended fabrics. TGA measurements with commercial cotton ball (solid line, *), residue after catalytic reaction with (solid line, ¤) and without (solid line, #) $NH_4HCO_3$, and starting material cloth (solid line, ^); In dashed lines: the corresponding DTG spectra. It is clear that the recovered cotton of catalysed reactions provides spectra similar to a commercial cotton ball, while textile from un-catalysed reaction is more similar to starting material.

**Figure 5B** shows infra-red spectroscopy of recovered cotton after depolymerisation of blended fabrics using $NH_4HCO_3$ (*), commercial cotton (¤), starting material (75% polyester, 25% cotton) (#) and residual after a reaction without the $NH_4HCO_3$ catalyst (^). It is shown that the IR spectrum of recovered cotton from the catalysed reaction is close to the spectrum for commercial cotton.

**Figure 6** shows the result of catalysed reaction on a mixture of a PET/cotton blended fabric and separately a number of other plastics/polymers. The reaction provides excellent yields of recovered cotton and BHET, while remaining plastics are not degraded and can be removed by filtration.

The present invention will now be described in more detail in the following.

**Detailed description of the invention**

Definitions

Prior to discussing the present invention in further details, the following terms and conventions will first be defined:

*Depolymerisation*

In the present context depolymerisation is the process of producing monomer units or precursors/derivatives thereof, from the polymer formed when these monomers are polymerised. As polymers are insoluble in most solvents depolymerisation happens partly in a heterogeneous mixture between polymer and reactants/solvent, which becomes more homogenous as the polymer is converted to oligomers and eventually single small molecules.

*Poly(ethylene)terephthalate*

In the present context polyethylene terephthalate, commonly abbreviated PET or PETE, is a polymer with a repeating unit of formula (a):

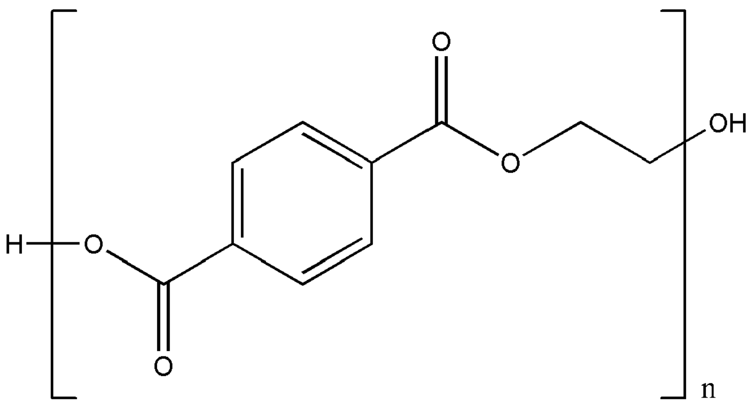

(a)

PET is the most common polymer of the polyester family. PET is a clear, strong, and lightweight plastic that is widely used for packaging foods and beverages. The basic building blocks of PET are ethylene glycol ($HO-CH_2CH_2-OH$) and terephthalic acid, which are combined to form the repeating unit of the polymer chain.

*Mixing*

In the present context mixing is the basic process of bringing starting materials, reactants, catalyst and any other materials into contact with each other while agitating the mixture, typically by mechanical stirring, although other types of agitation may be used. The mixture subject to mixing may in the broadest sense be a solid mixture, a suspension of solids in liquids, or a solution. In the present context the mixture obtained will often be a suspension of PET in a solvent with reactants either dissolved or also in suspension. As depolymerization occurs, the PET will slowly dissolve as oligomers and eventually monomers.

*Material comprising PET*

In the present context the material comprising PET may essentially be any material with any amount of PET comprised. As the present method may both be used to isolate non-PET material from PET or PET monomer precursors (or both). The invention is relevant for material comprising small amounts of PET (such as some blended fabrics, or waste with some fraction of PET) and virtually pure PET starting material (such as isolated end-of-use PET from e.g. municipal waste).

*Source of carbon dioxide*

$CO_2$ is a catalyst of the process of the present invention. Thus, in the present context, a source of carbon dioxide (COz) is any such source, that can provide $CO_2$ molecules under the reaction conditions of the present process. The source of $CO_2$ may be $CO_2$ as such, but preferably, the source is an easy to use solid or liquid pre-catalyst chemical, which releases $CO_2$ under the reaction conditions. The source of $CO_2$ may at the same time the source of other reactants, such as amine. Ammonium bicarbonate salt ($NH_4HCO_3$) is an example of a combined source of amine ($NH_3$) and $CO_2$.

*Source of amine*

Amine is a catalyst of the process of the present invention. Thus, in the present context, a source of amine is any such source, that can provide suitable amine molecules (such as $NH_3$) under the reaction conditions of the present process. The source of amine may be the amine as such, but preferably, the source is an easy to use solid or liquid pre-catalyst chemical, which releases the amine under the reaction conditions. Ammonia derivatives are also suitable sources of amine. The source of amine may at the same time the source of other reactants, such as COz. Ammonium bicarbonate salt ($NH_4HCO_3$) is an example of a combined source of amine ($NH_3$) and $CO_2$.

*Amine*

In the present context, the amine is any amine capable of catalyzing the depolymerization reaction (transesterification) of the present invention. Nucleophilic and/or basic amines are preferred to catalyze the process.

*Bis(hydroxyethyl)terephthalate (BHET)*

Bis(hydroxyethyl)terephthalate, abbreviated as BHET is an important industrial chemical, which is used as a precursor monomer of PET, but also has other applications. Bis(hydroxyethyl)terephthalate is a compound of formula (b):

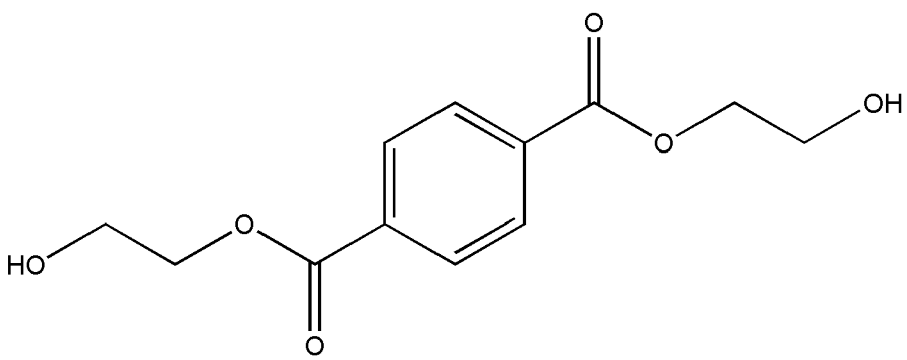

(b)

*Textile*

In the present context textile is any textile that may be combined with polyester fabric such as PET fabrics. Common textiles in this category includes cotton and viscose. Textile may also comprise other materials that are compatible or do not interfere with the process of the present invention. The textiles are often polymers, which are either natural (cotton, hemp) or synthetic (other polyesters, polyurethane, viscose, and others).

*Blended fabric*

In the present context a blended fabric is a fabric comprising two or more fabrics types, i.e. PET and one or

more textiles as defined above. Other materials may also be comprised (e.g. leather, rubber, metal (zippers, buttons), as long as they are not detrimental to the process of the present invention.

The present inventors have witnessed that $CO_2$ can be an active, highly accessible, inexpensive, traceless and safe catalyst for organic transformation. Also, without being bound to theory, the presence of nucleophilic amines under transamidation conditions suggested the role of amines and $CO_2$ in the activation of carbonyl functional groups: a nucleophilic and basic amine together with Lewis- and Brøndsted acidic $CO_2$ (in the form of carbonic acid) thus mediating dual catalysis. Therefore, the inventors report their investigation on transesterification of esters, PET bottles, PET-cotton blended fabrics to illustrate the advantage of catalytic CO2 together with amine as an additional base catalyst (Figure 1B).

Thus, a first aspect of the present invention is a process for chemical depolymerisation of poly(ethylene)terephthalate (PET) comprising the steps of:

e) providing a material comprising PET;

f) mixing the material comprising PET from step a) with a source of carbon dioxide, a source of amine, and a compound of formula (I):

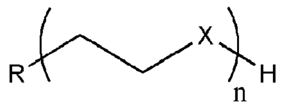

(I);

g) heating the mixture obtained in step b) to a temperature in the range of 130°C to 220°C;

h) obtaining a product comprising a compound of formula (II):

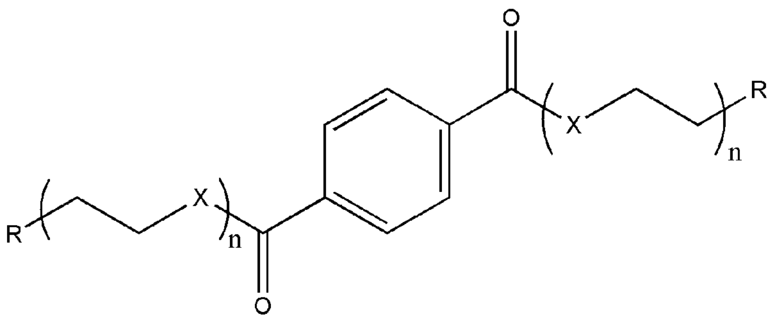

(II);

wherein

R is selected from the group consisting of OH and $N(R^1)_2$,

X is selected from the group consisting of O and $NR^2$,

$R^1$ and $R^2$ are independently selected from the group consisting of H, methyl, ethyl, propyl, isopropyl, butyl, and isobutyl,

n is any integer in the range of 1-10.

PET-based polymers are mainly used in beverage bottles, while a third of the produced PET is blended with cotton-based fibers and viscose to produce fabrics for the textile industry. Thus, in an embodiment the material comprising PET of step a) is selected from the group consisting of plastic bottles, plastic packaging, and a textile comprising PET, preferably plastic bottles or a textile comprising PET.

Although the present process is useful for any amount of PET in the material comprising PET, for producing BHET, it is advantage that the starting material has a high PET content. Therefore, in one embodiment, the material comprising PET comprises at least 30% of PET, such as at least 40% of PET, such as at least 60% of PET, such as at least 80% of PET, such as at least 90% of PET, such as at least 95% of PET, most preferably at least 99% of PET.

Carbon dioxide and amine are the catalysts of the present process. Without being bound to theory, both these catalysts interact with the ester groups of the PET polymer and nucleophiles (alcohols and amines), to activate these for transesterifaction or amidation with the compound of formula (I).

In one embodiment, the source of amine is selected from the group consisting of ammonium, ammonia and 4-dimethylaminopyridine (DMAP), preferably ammonium.

Using a source of carbon dioxide is important for the optimal performance of the present invention. The source of carbon dioxide may preferably be in the form of a solid pre-catalyst, and in one embodiment the source of carbon

dioxide is selected from the group consisting of carbonate, bicarbonate and carbon dioxide, preferably bicarbonate.

In a preferred embodiment, the source of carbon dioxide and the source of amine is combined in a single source of carbon dioxide and amine. Said single source of carbon dioxide and amine may be selected from the group consisting of ammonium bicarbonate or ammonium carbonate, preferably ammonium bicarbonate. Both ammonium carbonate and ammonium bicarbonate are tested in example 2 and are both effective.

The present inventors employed ammonium bicarbonate salt ($NH_4HCO_3$) as an inexpensive pre-catalyst (10-25 mol% loading), which can easily decompose to COz, ammonia, and water at above 36 °C (eq. 1).

$$NH_4HCO_3 \xrightarrow{36^\circ C} CO_2 + NH_3 + H_2O \qquad \text{eq. 1}$$

These reaction conditions offer practical and mild conditions for all tested wastes and recycled to generate high purity BHET, while selectively preserving cotton fabrics in the case of textile wastes.

Sub-stoichiometric amount of catalyst is sufficient to effectively provide good yields in the present process, and thus the single source of carbon dioxide and amine may be added in catalytic amounts as compared to PET, such as in the range of 5-40 mol%, preferably 10-40 mol%, more preferably 15-30 mol%, most preferably 20-30 mol%.

The compound of formula (1) may be a polyethylene glycol (n = 2-10, X = O, R = OH), and may also be a hydroxyethyl amine [n = 1, X = O, R = $N(R^1)_2$], or a diamine [n = 1, X = $NR^2$, R = $N(R^1)_2$]. In a preferred embodiment, the compound of formula (I) is ethylene glycol (n = 1, X = O, R = OH). Ethylene glycol is added in excess and may also function as solvent and/or suspending agent for PET and other reactants. Ethylene glycol may be added in 5-40 mol equivalents to the PET, preferably 10-30, more preferably 15-30, most preferably 20-30 mol equivalents to the PET.

In yet another embodiment, the amine (stemming from the source of amine) is $NH_3$. As with carbon dioxide, ammonia becomes gaseous, facilitating easy removal from products, whereas using a metal-based catalyst such as $ZnCl_2$ result in metal impurity of the final product.

In the glycolysis of PET-based beverage bottles, a wide range of different temperatures are applicable, and may vary according to reactants used and other parameters such as the time of heating. Thus, in an embodiment, the temperature of step c) is within the range of 150°C to 200°C, preferably 160°C to 200°C, more preferably 170°C to 200°C, most preferably 180°C to 200°C. Since, cellulose of cotton, which can be a constitute of the blended fabrics comprising PET, begins to degrade at 200°C, an embodiment of the present invention relates to that the temperature of step c) does not exceed 200°C. In an embodiment, the reaction mixture is kept at the temperature of step c) for at least three hours, such as within the range of 3-24 hours, preferably at least 4 hours, more preferably at least 5 hours, most preferably at least 6 hours. In another embodiment, the reaction mixture is agitated, such as stirred, during at least step c).

The compounds of formula (II) may be varied between the most common PET precursor BHET, but also to PEGylated derivatives and amine/amide derivatives in accordance with formula (II) which may have other applications.

In one embodiment, $R^1$ is H or methyl, preferably H, and in another embodiment, $R^2$ is H or methyl, preferably H. In another embodiment, X is O and/or R is OH. In yet another embodiment, n is 1-8, such as 1-6, 1-4, 1-3, 1-2, preferably 1. In the most preferred embodiment, the compound of formula (II) is bis(hydroxyethyl)-terephthalate (BHET).

High yields of the compound of formula (II) may be achieved in the process of the present invention. Thus, in an embodiment, the yield of the compound of formula (II) is at least 60% as compared to PET, preferably at least 70%, more preferably at least 80%, most preferably at least 90% as compared to PET.

Using metal salts as a catalyst results in metal impurities in the final product, whereas the same impurities are not observed for the process of the present invention. Thus, in an embodiment, the product comprising the compound of formula (II) comprises trace metals or salts thereof in an amount of less than 1000 ppm, such as 500 ppm, 200 ppm, 100 ppm, such as 80 ppm, 50 ppm, 30 ppm, such as preferably 10 ppm.

After catalysis according to the present depolymerization process, $CO_2$ can easily be evaporated, while the amine catalyst is also easily removable using various approaches. As the method leaves no traces as a catalyst and works well below 200 °C, the present inventors envisioned that amine/$CO_2$-catalyzed glycolysis of PET could be a practical and scalable approach, particularly as the method is selective for depolymerization of PET from blended fabrics with e.g. cotton. Cotton-based materials pose a significant challenge in chemical recycling due to the labile glycosidic bonds.

Thus, another aspect of the present invention is a process for separating poly(ethylene)terephthalate (PET) and a textile from a blended fabric comprising PET and textile, said process comprising the steps of:

f) providing a blended fabric comprising textile and PET;

g) mixing the blended fabric from step a) with a source of carbon dioxide, a source of amine, and a compound of formula (I):

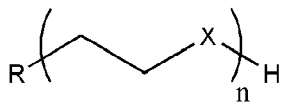

(I);

h) heating said mixture obtained in step b) to a temperature in the range of 130°C to 220°C;
i) obtaining a product comprising a compound of formula (II):

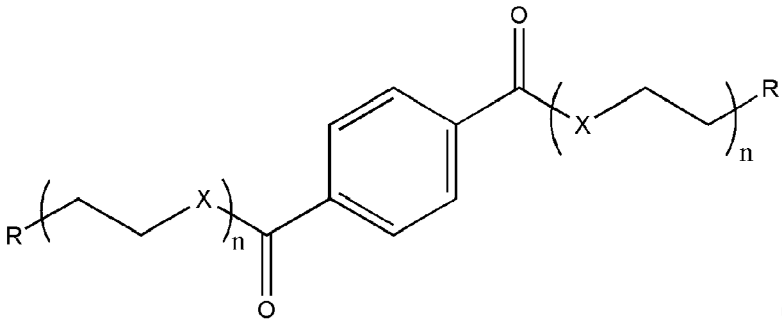

(II)

and textile;
j) separating the compound of formula (II) and textile of said product of step d);

wherein

R is selected from the group consisting of OH and $N(R^1)_2$,
X is selected from the group consisting of O and $NR^2$,
$R^1$ and $R^2$ are independently selected from the group consisting of H, methyl, ethyl, propyl, isopropyl, butyl, and isobutyl,
n is any integer in the range of 1-10.

Blended fabrics may be sourced from several industries including the clothing and furniture industries. Thus, in an embodiment, the blended fabric is selected from the group consisting of garments, furniture covers, carpets and curtains. The blended fabric comprising PET can contain varies other textiles. Hence, in an embodiment, the textile of step a) is selected from the group consisting of cotton, viscose, lyocell, polyurethane or a mixture thereof. The present process is able to depolymerize PET while keeping cotton fibres intact, and thus a preferred textile is cotton. In another embodiment, the ratio between the textile and PET is in the range of 10:1 to 1:10, such as 3:1 to 1:3, 2:1 to 1:2, preferably 3:1 to 1:1, most preferably 2:1 to 1:1. The present process is highly selective and other components may be present apart from textile and PET. That said, separation of BHET and textile is generally easier in the absence of further components, and thus in one embodiment, the blended fabric consists of PET and textile, i.e. essentially without the presence of further components.

Since the cellulose fibres of cotton, which can be a constituent of the blended fabrics comprising PET, begins to degrade at 200°C, an embodiment of the present invention relates to, that the temperature of step c) does not exceed 200°C.

The optimised yields of textile in the process of the invention are good due to the relatively mild reaction conditions. Thus, in a preferred embodiment, the textile yield is at least 60%, preferably at least 70%, more preferably at least 80%, most preferably at least 90%. Another advantage of the present process is the lack of metal catalyst, which may often reside in the textile and PET after the process. Therefore, the product comprising the compound of formula (II) and/or the textile may preferably comprises trace metals or salts thereof in an amount of less than 1000 ppm, such as 500 ppm, 200 ppm, 100 ppm, such as 80 ppm, 50 ppm, 30 ppm such as preferably 10 ppm.

There are a number of ways known to the skilled person to separate the textile and the compound of formula (II) according to the present process. These processes may be adjusted and optimised also according to whether further components are present, and the physical characteristics of the compound of formular (II). Often the compound of formula (II) will be dissolved in the solvent used (e.g. the compound of formula (I)) and therefore filtration is possible. Hence, in an embodiment, the compound of formula (II) and the textile are separated using filtration, preferably hot filtration.

The textile may be further purified a number of ways known to the skilled individual or adapted therefrom. In an embodiment, the textile is further purified by washing with a composition selected from water, organic solvent, surfactants, and any mixture thereof. More specifically, the cotton residual obtained through the process of the present invention was washed with water, ethanol, and diethyl ether. Hence, in an embodiment, the textile is washed with water, ethanol and diethyl ether. The compounds of formula (II) may be further isolated and purified using standard methods for small molecules, such as e.g. BHET, which may be crystallized. Thus, in an embodiment, the compound of formula (II) is further purified using a method selected from the group consisting of crystallization and column chromatography, preferably crystallization. In yet another embodiment, the blended fabric is added to the reaction as part of a composition also comprising further polymers.

It should be noted that embodiments and features described in the context of one of the aspects of the present invention also apply to the other aspects of the invention. In particular, the embodiments described for the first aspect relating to a process for chemical depolymerisation of PET also apply *mutatis mutandis* to the aspect related to separating (PET) and a textile from a blended fabric.

All patent and non-patent references cited in the present application, are hereby incorporated by reference in their entirety.

The invention will now be described in further details in the following non-limiting examples.

## EXAMPLES

### Example 1 - $NH_4HCO_3$-catalyzed transesterification reactions with ethylene glycol and derivatives thereof

*Aim of study*

The aim of this study was to establish a metal-free transesterification protocol by investigating the compound yields obtained by different transesterification reactions using ethylene glycol (Figure 2A) and derivatives thereof (Figure 2B). The reactions were catalysed by $NH_4HCO_3$ or performed without a catalyst under $N_2$ atmosphere to investigate whether $NH_4HCO_3$ was a suitable catalyst for the transesterification reactions.

*Materials and methods*

A vial (8 ml) equipped with a magnet was charged with ester (1.0 mmol), alcohol (10.0 mmol, 10 equiv.) and ammonium bicarbonate (12-25 mol%). The vial was stirred at 130-150 °C and conversion was detected by analyzing aliquot samples by $^1$H NMR spectroscopy. After full conversion, the reaction mixture was directly subjected to silica gel chromatography to isolate the desired product.

*Results*

Ethylene glycolysis was tested with model substrates (methyl- and ethylesters, **1**) to confirm the applicability of the process in depolymerization (Figure 2A). Catalytic amounts of $NH_4HCO_3$ (12-25 mol%) enabled a transesterification reaction of methyl- or ethylesters (**1**) with ethylene glycol (**2a**, 10 equiv.) under mild reaction conditions (80-150 °C) affording the desired glycolysis products (**3**). The tested transesterification substrates were not reactive in the absence of the catalyst (percentages in grey in figure 2A); only trace amounts of products were observed under nitrogen atmosphere (up to 28 % conversion detected by $^1$H NMR spectroscopic analysis of reaction mixtures). Under catalytic conditions, good to high yields ranging from 19% to 91% (percentages in black in figure 2A) of ethylene glycol esters were obtained regardless of the electronic properties of the substrates. Diethyl terephthalate, **1m,** employed as a model substrate for PET depolymerization, and the catalytic conditions afforded a reasonable isolate yield (57%) of BHET (**3m**) at 80°C while producing a mono-substituted product (36%) (**3m'**), implying approximately quantitative conversion of substrate **1m** (full conversion by $^1$H NMR analysis). Encouraged by this result, the inventors further investigated whether the $NH_4HCO_3$ catalyst where applicable in the transesterification of other ethylene glycol derivatives namely oligo(ethylene glycol) **2a-2k,** which can produce thermoresponsive polymers (Figure 2B). With higher catalyst loading at an elevated temperature (25 mol%, 150 °C), the methodology afforded the selective mono-esterification products with moderate isolated yields (up to 55% isolated yield (**2c**)). 2-(Dimethylamino)ethan-1-ol (**2h**) afford moderate yield of the transesterification product. On the contrary, monomethyl ether equivalents **(2e-2g)** showed no substantially lower conversion, manifesting the importance of the diol functional group in the catalytic transesterification reaction: polarity, hydrogen bonding donor ability, dipole moments are important in the activation of carbonyls.

*Conclusion*

The transesterification of methyl- and ethylesters with ethylene glycol was more effective when $NH_4HCO_3$ was used as a catalyst i.e. the compound yield of the different model substrates were higher (19-91%) as compared to the

compound yield without a catalyst under $N_2$ atmosphere (<1-28%). In addition, transesterification could be performed using derivatives of ethylene glycol specifically oligo(ethylene glycol) and 2-(Dimethylamino)ethan-1-ol.

**Example 2** - **Optimization of reaction conditions for glycolysis of PET-based beverage bottles**

*Aim of study*

After establishing a metal-free transesterification protocol, the inventors turned their attention to PET substrates to generate monomer BHET. Further optimization of glycolysis was necessary to provide practical reaction conditions to access high purity BHET. It is important to note that minor metal impurities derived from catalysts and pre-treatment processes impose lower quality of recycled PET thus lowering the value of the whole recycling process. The aim of this study was therefore to optimize the reaction conditions for glycolysis of PET beverage bottles by changing the equivalents of ethylene glycol and $CO_2$, the catalyst used, and the reaction temperature.

*Materials and methods*

A vial (8 ml, or 250 mL round bottom flask when the reaction is scaled up) equipped with a magnet was charged with PET substrate (2.0 mmol), ethylene glycol (25 equiv.) and a catalyst. The vial was stirred at 150 - 200 °C for 6-24 h. The $^1$H NMR-yields were detected with aliquot samples in DMSO-$d_6$ with 1,3,5-trimethoxybenzene as an internal standard. To determine conversion and yields of BEHT, reaction mixture was filtered to remove oligomers, unreacted and remaining PET substrate. To the filtrate, water was added to precipitation out BHET at 4 °C. The crystalline BHET was washed with cold water and diethyl ether to remove excess ethylene glycol, and dried to determine the isolated yield of BHET.

*Results*

The inventors conducted an intensive optimization of reaction conditions with drinking bottles as depolymerization substrate for glycolysis with various catalytic systems with and without the use of $CO_2$ (Table 1). The inventors first confirmed that no background glycolysis was performed without catalysts (150-200 °C, table 1; entry 1). The use of $CO_2$ in catalytic amounts (15 -30 mol%) and as a reaction atmosphere (1 atm) showed no effects without a catalyst (table 1; entry 2). The addition of the catalyst DMAP (30 mol%) afforded a higher yield of BHET (44%, table 1; entry 4) than when DMAP was employed without $CO_2$ (24%, table 1; entry 3). This set of experiments confirmed the positive effect of $CO_2$ in depolymerization of PET using DMAP as a catalyst (30 mol%).

**Table 1:**

| Entry | Ethylene glycol (equiv.) | $CO_2$ gas (mol%) | Catalyst (mol%) [amine or amine + $CO_2$ source] | T (°C) | Yield of BHET[a] |
|---|---|---|---|---|---|
| 1[b] (ref) | 20 | 0 | - | 150-200 | No reaction |
| 2[b] (ref) | 20 | 15 | - | 150 | No reaction |
| 3 (ref) | 20 | 0 | DMAP, 30 mol% | 150 | 24% |
| 4[b] | 20 | 30 | DMAP, 30 mol% | 150 | 44% |
| 5 | 10 | 30 | DMAP, 30 mol% | 180 | 40% |
| 6 | 10 | 0 | $NH_4HCO_3$, 10 mol% | 180 | 65% |
| 7 | 25 | 0 | $NH_4HCO_3$, 25 mol% | 180 | 84% |
| 8 | 25 | 0 | $NH_4HCO_3$, 25 mol% | 180 | >70% (50 mmol) |
| 9 (ref) | 25 | 0 | - | 180 | 8% |
| 10 (ref) | 20 | 0 | $Zn(OAc)_2$, 30 mol% | 180 | 15% |
| 11 (ref) | 25 | 0 | $Zn(OAc)_2$, 25 mol% | 180 | 84% |

(continued)

| Entry | Ethylene glycol (equiv.) | $CO_2$ gas (mol%) | Catalyst (mol%) [amine or amine + $CO_2$ source] | T (°C) | Yield of BHET[a] |
|---|---|---|---|---|---|
| 12 | 25 | 0 | $(NH_4)_2CO_3$, 25 mol% | 180 | 87% |
| [a]*Yield of BHET was determined by* $^1$*H NMR spectroscopy in DMSO-*$d_6$ *with 1,3,5-trimethoxybenzene as an internal standard.* [b]*Reaction time was extended to 24 h.* | | | | | |

The inventors further optimized the equivalent of ethylene glycol (EG) used in the reaction; a BHET yield of 40% was obtained with 10 equivalents of EG ( table 1; entry 5). Next, the inventors further optimized the conditions aiming for a higher conversion to BHET, assuming the recovery of EG is trivial due to the high boiling point of EG. When the inventors replaced the DMAP/$CO_2$ combination with $NH_4HCO_3$, higher yields were obtained with 10-25 equivalents of EG (table 1; entries 6 and 7). This optimization led to an 84% yield of BHET highlighting the exclusive selectivity towards the monomer at 180 °C after 6 hours of reaction time. This reaction was further tested in a larger scale (50 mmol, 9.6 grams of waste PET bottles, table 1; entry 8) affording high selectivity and isolated yield of high purity BHET (>70% yield, up to 99.8% HPLC purity) without any sophisticated purification since all catalytically active species were evaporated. The advantage of this protocol is that the BHET product was crystalized from the filtrate after hot filtration by adding cold deionized water (6%, V/V). Considering that vacuum distillation was required after methanolysis of PET, this method showcased advantages in terms of energy economy of $CO_2$-catalyzed catalytic glycolysis. Once again, the same reaction in the absence of the catalyst showed negligible conversion (7% by $^1$H NMR spectroscopic analysis of the reaction mixture), highlighting the utility of $CO_2$ and ammonia in catalytic glycolysis. Ammonium bicarbonate ($NH_4HCO_3$) has been utilized as a catalyst in a Knoevenagel condensation, which would decompose at the high reaction temperatures to generate $NH_3$ and $CO_2$ *in situ* to act as acid and base catalysts (see eq. 1 above).

Since, metal catalyst have previously been used in PET glycolysis, representative Zn-based Lewis acidic catalysts were tested herein. $Zn(OAc)_2$ (table 1; entries 10 and 11) showed low to good yields of BHET, however, elemental analysis of the isolated BHET showed significant metal impurity (data not shown), presumably due to the residual zinc. The effect of ammonium carbonate $(NH_4)_2CO_3$ (table 1; entry 12) was comparable to that of the ammonium bicarbonate catalyst, affording good yields of BHET (87% yield, table 1; entry 12), while strong inorganic bases (KOH, $Na_2CO_3$, and NaOH) led to the contamination of BHET with the hydrolysis product, terephthalic acid, TPA (data not shown).

The reactions were repeated at a larger scale (2-50 g PET) with a fixed equivalence of ethylene glycol (25 equiv.) at 180 °C for 6 h (unless otherwise indicated) using the catalysts listed in table 2. Isolated yields were also determined for BHET.

**Table 2:**

| *Entry* | *PET (9)* | *Catalyst (mol%)* | *Conversion (%)* | *Yield of BHET (%)* |
|---|---|---|---|---|
| *1[b] (ref)* | *0.2 g* | *$CO_2$ (15-30 mol% or 1 atm)* | *not detected* | *0%* |
| *2 (ref)* | *2 g* | *$Zn(OAc)_2$ (25 mol%)* | *44%* | *40%* |
| *3 (ref)* | *2 g* | *$ZnCl_2$ (25 mol%)* | *75%* | *52%* |
| *4 (ref)* | *2 g* | *DMAP (30 mol%)* | *63%* | *46%* |
| *5* | *2 g* | *DMAP/$CO_2$ (30 mol%)* | *81%* | *69%* |
| *6* | *9 g* | *$NH_4HCO_3$ (10 mol%)* | *65%* | *51%* |
| *7* | *9 g* | *$NH_4HCO_3$ (25 mol%)* | *63%* | *52%* |
| *8 (ref)* | *2 g* | *$NH_4OH$ (25 mol%)* | *27%* | *26%* |
| *9* | *50 g* | *$NH_4HCO_3$ (25 mol%)* | *88%* | *64% (32 g)* |
| [a]*Reaction conditions: PET bottle substrate (0.2-50 g); a catalyst and ethylene glycol were added with a magnetic stirring bar, and the vial was heated for 6 h; Conversion (%) = ($W_{oligomers+BHET}/W_{PET\ bottles}$)×100, W = weight. The reaction mixture was diluted with water to precipitate oligomers and BHET, which was crystalized to determine the yield of isolated BHET.* [b]*Reaction time extended to 24 h.* | | | | |

There was no background glycolysis in the absence of catalysts, even at prolonged reaction times (up to 24 h, at 150-200 °C). The use of $CO_2$ in catalytic amounts (15-30 mol%) and as a reaction atmosphere (1 atm) showed no effects (Table 2; Entry 1). Well-known Zn-based Lewis acidic catalysts for glycolysis, e.g., $Zn(OAc)_2$ and $ZnCl_2$ (Table 2; Entries 2 and 3), showed low to good conversion to BHET - 44% and 75%, respectively. However, an elemental analysis of the isolated BHET using the same purification sequence (hot filtration and crystallization) often showed various degrees of metal impurity (data not shown), presumably because of the residual zinc ions. The employment of organocatalyst DMAP (30 mol%) under $N_2$ conditions afforded 63% conversion and 46% isolated yield of BHET (Table 2; Entry 4). The addition of catalytic amounts of $CO_2$ (30 mol%) resulted in higher conversion and yield of BHET (Table 2; Entry 5) than when DMAP was used alone, confirming the positive effect of $CO_2$ in glycolysis.

The catalytic $NH_4HCO_3$ showed an activity comparable to that of metal catalysts (Table 2; Entries 6 and 7), achieving a >50% isolated yield of BHET from a single crystallization step. A hot filtration, followed by a single crystallization operation with cold DI water (6%, V/V), afforded BHET in high purity (up to 99.8% HPLC purity), since all catalytic species and glycol could easily be removed (data not shown). The effect of ammonia as a sole catalyst (in water and methanol), without $CO_2$, was inferior to that of $NH_4HCO_3$ (Table 2; Entry 8) confirming dual catalysis with $CO_2$ and ammonia is critical to achieve optimal performance in catalytic glycolysis. Under optimized conditions, a larger scale (260 mmol, 50 grams of waste PET bottle substrate), quantitative conversion of PET with 51% selectivity toward BHET in 64% isolated yield was achieved (Table 2; Entry 9). It should be noted here that, under the tested catalytic conditions, there was no formation of cyclic carbonate or urea from the catalytic species and ethylene glycol.

*Conclusion*

Dual catalysis with ammonia or ammonium and $CO_2$ is critical to achieve an optimal performance in catalytic glycolysis of PET-based beverage bottles. The $ZnCl_2$ catalyst was also effective in the depolymerisation of PET, however, a significant metal impurity was observed by elemental analysis of the isolated BHET, presumably due to the residual zinc. Hence, a source of ammonium or ammonia and carbon dioxide are very effective catalysts for depolymerisation of PET yielding high percentages of "pristine" PET, i.e. PET with similar or the same quality as non-recycled PET.

**Example 3** - **Glycolysis of PET in blended fabrics**

*Aim of study*

After successfully applying the method in chemical recycling of PET waste bottles (example 2), the inventors attempted to test the reaction conditions for blended fabrics. The aim of this study was therefore to investigate different reaction conditions for depolymerisation of PET in blended fabrics and the concomitant recovery of cotton in a PET:cotton blended fabric.

*Materials and methods*

A vial (40 ml) equipped with a crossed magnet was charged with textile substrate (2.0 g of polyester), ethylene glycol (25 equiv., 15.8 mL) and ammonium bicarbonate (25 mol%). The vial was stirred at 160 - 190 °C for 6-24 h. The control experiments were performed without ammonium bicarbonate but under a $N_2$ atmosphere. The $^1H$ NMR-yields were detected by analyzing aliquot samples in DMSO-$d_6$ with 1,3,5-trimethoxybenzene as an internal standard. The reaction mixture was filtered while still hot to remove solid residues and insoluble oligomers, and then washed with 5 mL of water. The combined filtrate was cooled down to 4 °C, to crystalize BHET. The purity of BHET was confirmed by HPLC (conditions: column: Kromasil 5-AmyCoat (4.6 x 250 mm), iso-propanol/n-heptane (60/40, v/v), 0.5 mL/min, 8.9 MPa, detector A: 220 nm, detector B: 254 nm.)

*Results*

The inventors identified $NH_5HCO_3$ as an optimal catalyst affording high $^1H$ NMR yields of BHET from blended fabric (62% polyester, 33% viscose, 5% elastane wt% based on the tag of the merchandise) and quantitative yields of recovered cotton (Figure 3A). Time-dependent yields of BHET was plotted over 6 h of a reaction illustrating the positive effect of catalysis (Figure 3B). In the absence of the catalyst and under a $N_2$ atmosphere, lower conversion to BHET was recorded. Higher reaction temperatures (at 190 °C, full circles) showed higher yields of BHET under catalytic and non-catalytic conditions as compared to lower temperatures (at 180°C), while the reaction with the catalyst (25 mol%) was far superior achieving a higher yield of BHET as compared to the reaction without the catalyst (Figure 3B).

Next, recovery of cotton from PET/cotton blended fabric was investigated. The labile nature of glycosidic bonds of cotton materials should be taken into consideration when selective PET depolymerization is a desired process in

parallel. To avoid acid and base-mediated hydrolysis of cotton materials, it is important to mediate the depolymerization under mild and neutral conditions to afford recycled cotton for textile industry and others. The inventors analyzed reaction mixtures under catalytic conditions to inspect polymer degradation degrees over time. Also the recovered cotton residues were analyzed by optical microscope, thermal gravimetric analysis (TGA), infrared spectroscopy, solid-state NMR spectroscopy and elemental analysis. During large-scale glycolysis of PET/cotton blended fabrics both BHET and cotton were recovered (Figure 4A). For the glycolysis of a T-shirt containing 75% PET with a 71% conversion, a yield of 47% BHET and 92% cotton and viscose were recovered. For the glycolysis of a sofa cover (213 g of textile with 47% polyester and 53% cotton), a yield of 62% BHET and 94% cotton was recovered. The residues recovered from the reaction without catalyst showed identical signals compared to the starting material, which is in accordance with the NMR yield detected by $^{1}H$ NMR spectroscopy (Figure 4B). On the other hand, the residual sample obtained from the reaction mixture with the catalyst displayed similar signals compared to a commercial cotton ball without signals detected from 130 to 163 ppm (aromatic signals and carbonyls from PET) indicating the full degradation of PET while displaying $^{13}C$ signals responsible for cotton (Figure 4B). It is clear from the optical microscope images of figure 4C that cotton recovered using the $NH_4HCO_3$ catalyst is more comparable to the images of the substrate than when the cotton is recovered with $Zn^{2+}$ glycolysis i.e. the recovered cotton of the present invention is more "pristine" than the cotton recovered using $Zn^{2+}$ glycolysis (Figure 4C). Considering the further utilization of cotton and BHET, the inventors confirmed traceless $NH_4HCO_3$ showcased more practical application in large scale, when compared to heavy metal catalysis, for example $ZnCl_2$, due to the catalyst vestigial, which was detected by the elemental analysis of the recovered cotton and BHET (data not shown). In addition, the inventors noticed that the selective depolymerisation was realized using $NH_4HCO_3$, while heavy metal catalysts would cause cotton degradation. Microscopy at 80 times revealed that residual recovered after reaction with catalyst showed only cotton fiber similar to that of commercial cotton ball (Figure 4D).

According to the thermal gravimetric analysis, identical one-step mass losses were detected with the commercial cotton ball (solid line, *) and the residue isolated after catalytic reaction (solid line, ¤), suggesting the selective degradation of polyester from the textile (Figure 5A). Reasonably, similar two-step mass losses were observed for the starting material (solid line, ^) and the residue recovered after glycolysis without the catalyst $NH_4HCO_3$ (solid line, #). The corresponding DTG spectrums are shown in dash lines. The first mass loss (25% to 30% respectively) could be assigned to the decomposition of cotton (15% viscose and 10% cotton). The infra-red spectrum of recovered cotton (*) is close to the spectrum for commercial cotton (¤), whereas the IR spectrum for residual recovered without the $NH_3HCO_3$ catalyst (^) resembles the spectrum of the starting material (#) (Figure 5B).

*Conclusion*

The process of the present invention can be used to effectively recover BHET and cotton from PET/cotton blended fabrics, with no trace metals in the products and high yields.

**Example 4** - **Depolymerisation of PET in blended fabrics with additional components present in the material comprising PET**

*Aim of study*

To determine the selectivity of the reaction towards PET in the presence of various other components, and to see whether BHET and textile can be isolated from the resulting mixture.

*Materials and methods*

Trash mimicking glycolysis with 42.7 g of blended fabric (PET: cotton= 75:25 wt% based on the tag of the merchandise) and ethylene glycol (25 equiv.) were performed by following the procedure described in Example 3 with the following components further added to the reaction mixture: polypropylene, lego, polyyurethane, polystyrene, acrylonitrile butadiene rubber (NBR), acrylonitrile butadiene styrene (ABS), hair, polyamide (PA), polyethylene (PE), styrene-ethylene-butylene-styrene (SEBS), polyoxomethylene (POM), polyvinylchloride (PVC), polypropylene (PP), polystyrene (PS), and polyurethane (PU).

*Results*

The inventors mixed several plastic materials (PVC, PP, PE, polyamide, PS, ABS, POM, and SEBS, PU) to mimic realistic, daily use, plastic waste, and demonstrate the selective glycolysis of PET (Fig. 7). Under standard reaction conditions, we obtained a 23% yield of BHET after recrystallization, and of 75 % of recovered cotton, while the other plastic materials were collected with some deformation, but without clear signs of chemical depolymerization. The for-

mation of pre-catalyst $NH_4HCO_3$ during the course of the reaction was confirmed, and the catalyst recovered by simple sublimation.

*Conclusion*

The process of the present invention can be used selectively in the presence of several other types of waste and chemicals, and textile and BHET can be isolated.

**References**

- M.Ghaemy et al., Polymer Degradation and Stability, Volume 90, Issue 3, December 2005, Pages 570-576
- WO 2015/056377


**Claims**

**1.** A process for chemical depolymerisation of poly(ethylene)terephthalate (PET) comprising the steps of:

i) providing a material comprising PET;
j) mixing the material comprising PET from step a) with a source of carbon dioxide, a source of amine, and a compound of formula (I):

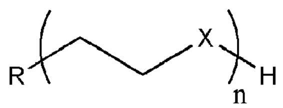
(I);

k) heating the mixture obtained in step b) to a temperature in the range of 130°C to 220°C;
l) obtaining a product comprising a compound of formula (II):

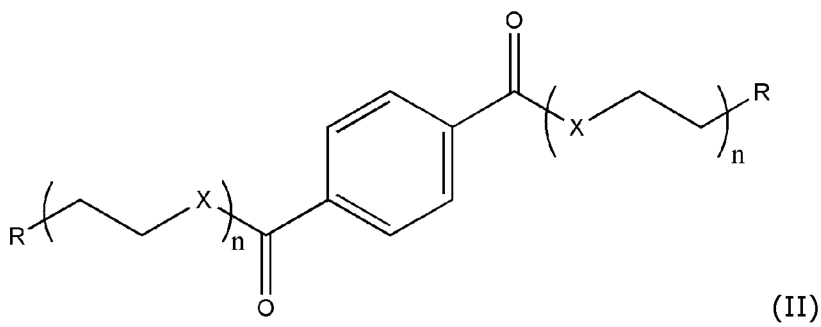
(II);

wherein

R is selected from the group consisting of OH and $N(R^1)_2$,
X is selected from the group consisting of O and $NR^2$,
$R^1$ and $R^2$ are independently selected from the group consisting of H, methyl, ethyl, propyl, isopropyl, butyl, and isobutyl,
n is any integer in the range of 1-10.

**2.** The process according to claim 1, wherein the source of amine is selected from the group consisting of ammonium, ammonia and 4-dimethylaminopyridine (DMAP), preferably ammonium.

**3.** The process according to any one of the preceding claims, wherein the source of carbon dioxide is selected from the group consisting of carbonate, bicarbonate and carbon dioxide, preferably bicarbonate.

**4.** The process according to any one of the preceding claims, wherein the source of carbon dioxide and the source of amine is combined in a single source of carbon dioxide and amine.

**5.** The process according to any one of the preceding claims, wherein the single source of carbon dioxide and amine is ammonium bicarbonate or ammonium carbonate, preferably ammonium bicarbonate.

**6.** The process according to any one of the preceding claims, wherein the temperature of step c) is within the range of 150°C to 200°C, preferably 160°C to 200°C, more preferably 170°C to 200°C, most preferably 180°C to 200°C.

**7.** The process according to any one of the preceding claims, wherein X is O.

**8.** The process according to any one of the preceding claims, wherein R is OH.

**9.** The process according to any one of the preceding claims, wherein n is 1-8, such as 1-6, 1-4, 1-3, 1-2, preferably 1.

**10.** The process according to any one of the preceding claims, wherein the compound of formula (II) is bis(hydroxyethyl)terephthalate (BHET).

**11.** The process according to any one of the preceding claims, wherein the product comprising the compound of formula (II) comprises trace metals or salts thereof in an amount of less than 1000 ppm, such as 500 ppm, 200 ppm, 100 ppm, such as 80 ppm, 50 ppm, 30 ppm, such as preferably 10 ppm.

**12.** A process for separating poly(ethylene)terephthalate (PET) and a textile from a blended fabric comprising PET and textile, said process comprising the steps of:

k) providing a blended fabric comprising textile and PET;
l) mixing the blended fabric from step a) with a source of carbon dioxide, a source of amine, and a compound of formula (I):

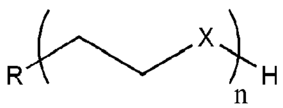

(I);

m) heating said mixture obtained in step b) to a temperature in the range of 130°C to 220°C;
n) obtaining a product comprising a compound of formula (II):

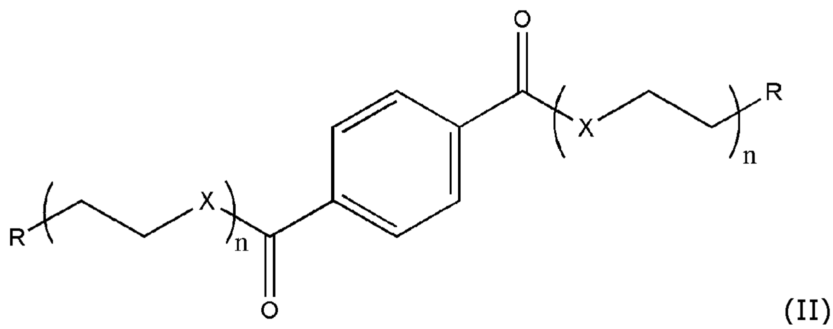

(II)

and textile;
o) separating the compound of formula (II) and textile of said product of step d);

wherein

R is selected from the group consisting of OH and $N(R^1)_2$,
X is selected from the group consisting of O and $NR^2$,
$R^1$ and $R^2$ are independently selected from the group consisting of H, methyl, ethyl, propyl, isopropyl, butyl, and isobutyl,

n is any integer in the range of 1-10.

**13.** The process according to claim 12, wherein the textile of step a) is selected from the group consisting of cotton, viscose, lyocell, polyurethane or a mixture thereof, preferably cotton.

**14.** The process according to any one of claims 12-13, wherein the ratio between the textile and PET is in the range of 10:1 to 1:10, such as 3:1 to 1:3, 2:1 to 1:2, preferably 3:1 to 1:1, most preferably 2:1 to 1:1.

**15.** The process according to any one of claims 12-14, wherein the product comprising the compound of formula (II) and the textile comprises trace metals or salts thereof in an amount of less than 1000 ppm, such as 500 ppm, 200 ppm, 100 ppm, such as 80 ppm, 50 ppm, 30 ppm such as preferably 10 ppm.

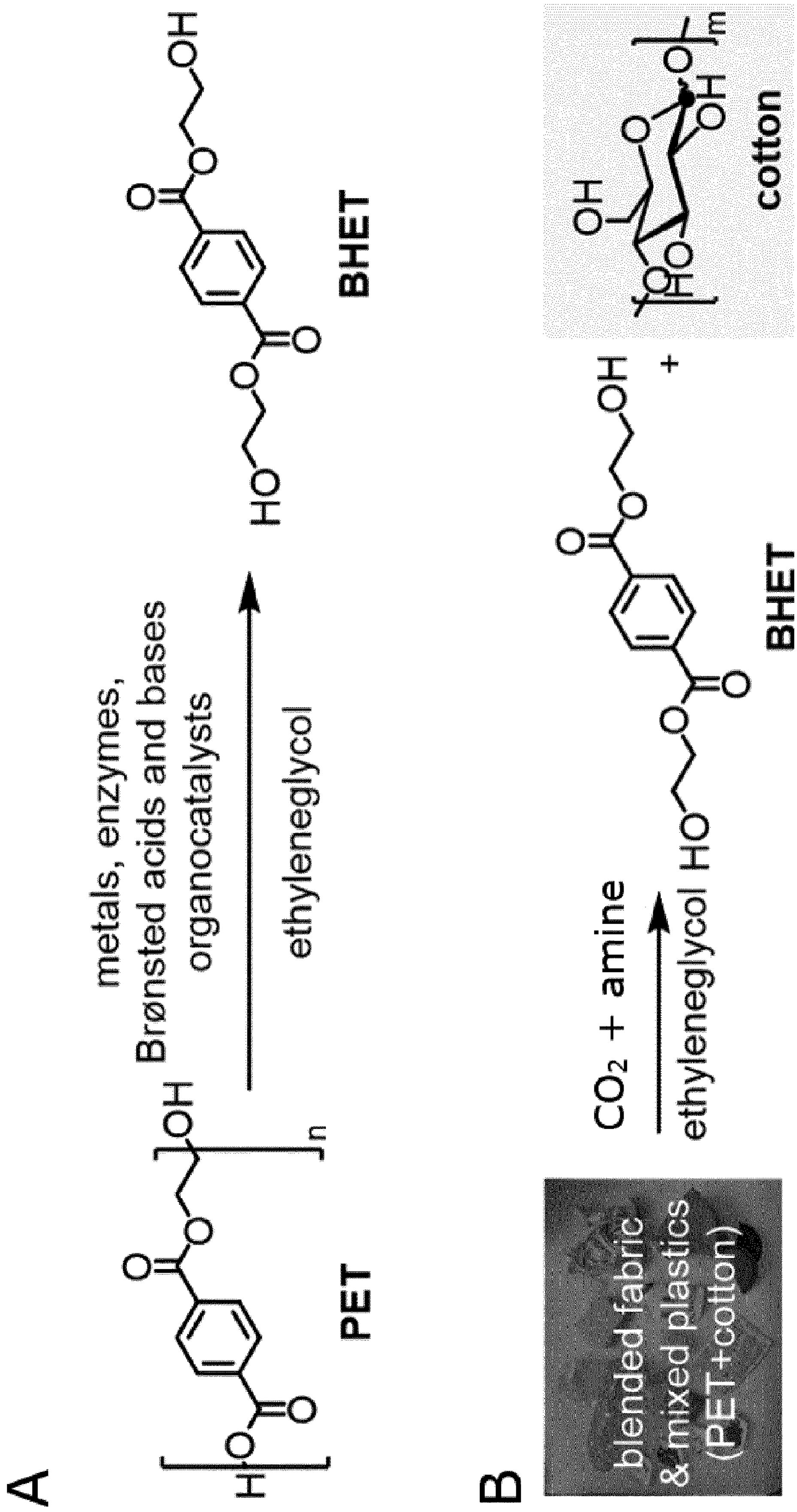
A
PET
metals, enzymes,
Brønsted acids and bases
organocatalysts
ethyleneglycol
BHET
B
blended fabric
& mixed plastics
(PET+cotton)
CO2 + amine
ethyleneglycol
BHET
cotton

Fig. 1

A

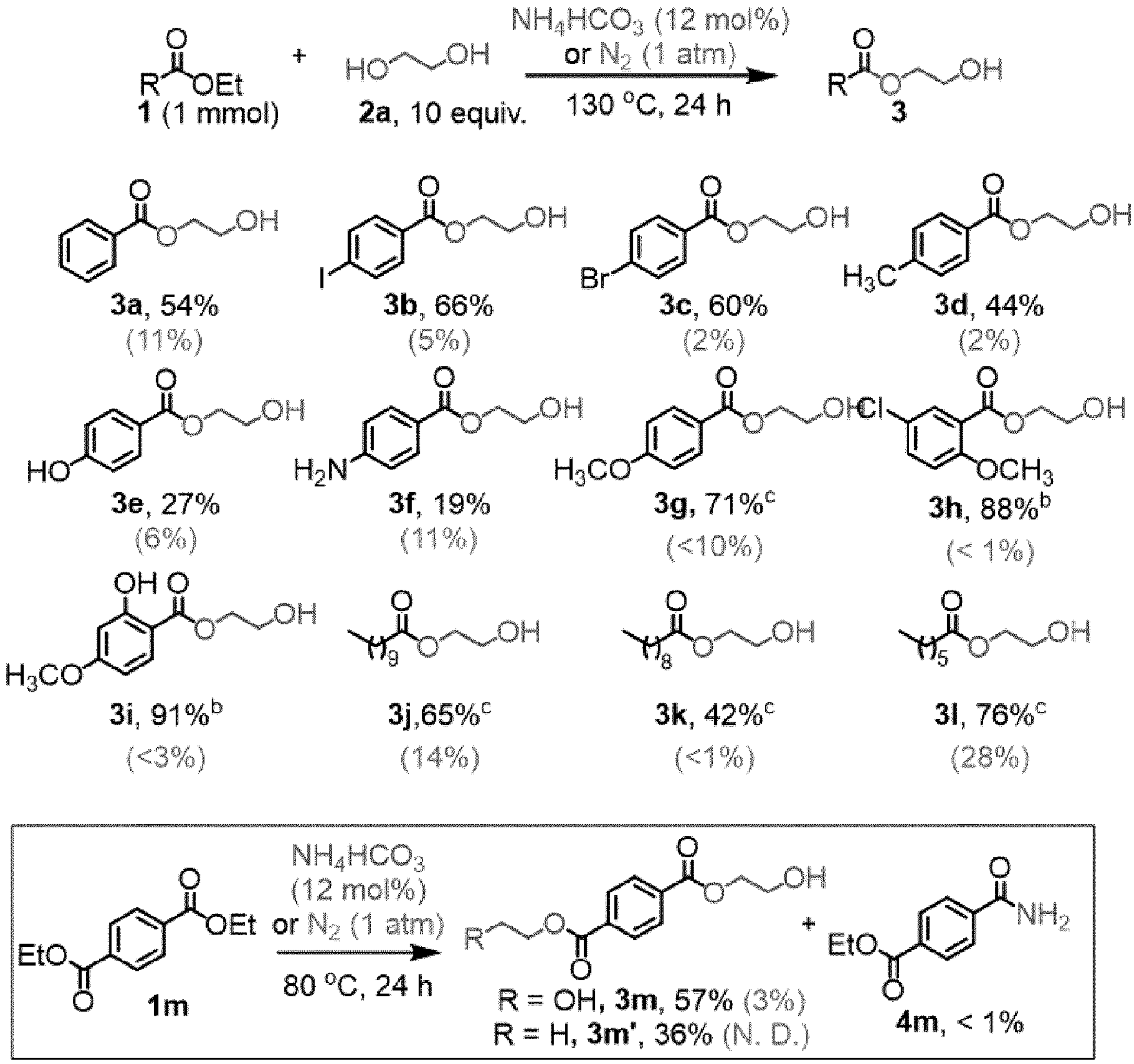
1 (1 mmol)
+
2a, 10 equiv.
$NH_4HCO_3$ (12 mol%)
or $N_2$ (1 atm)
130 °C, 24 h
3
3a, 54%
(11%)
3b, 66%
(5%)
3c, 60%
(2%)
3d, 44%
(2%)
3e, 27%
(6%)
3f, 19%
(11%)
3g, 71%[c]
(<10%)
3h, 88%[b]
(< 1%)
3i, 91%[b]
(<3%)
3j,65%[c]
(14%)
3k, 42%[c]
(<1%)
3l, 76%[c]
(28%)
1m
$NH_4HCO_3$
(12 mol%)
or $N_2$ (1 atm)
80 °C, 24 h
R = OH, 3m, 57% (3%)
R = H, 3m', 36% (N. D.)
+
4m, < 1%

Fig. 2A

B

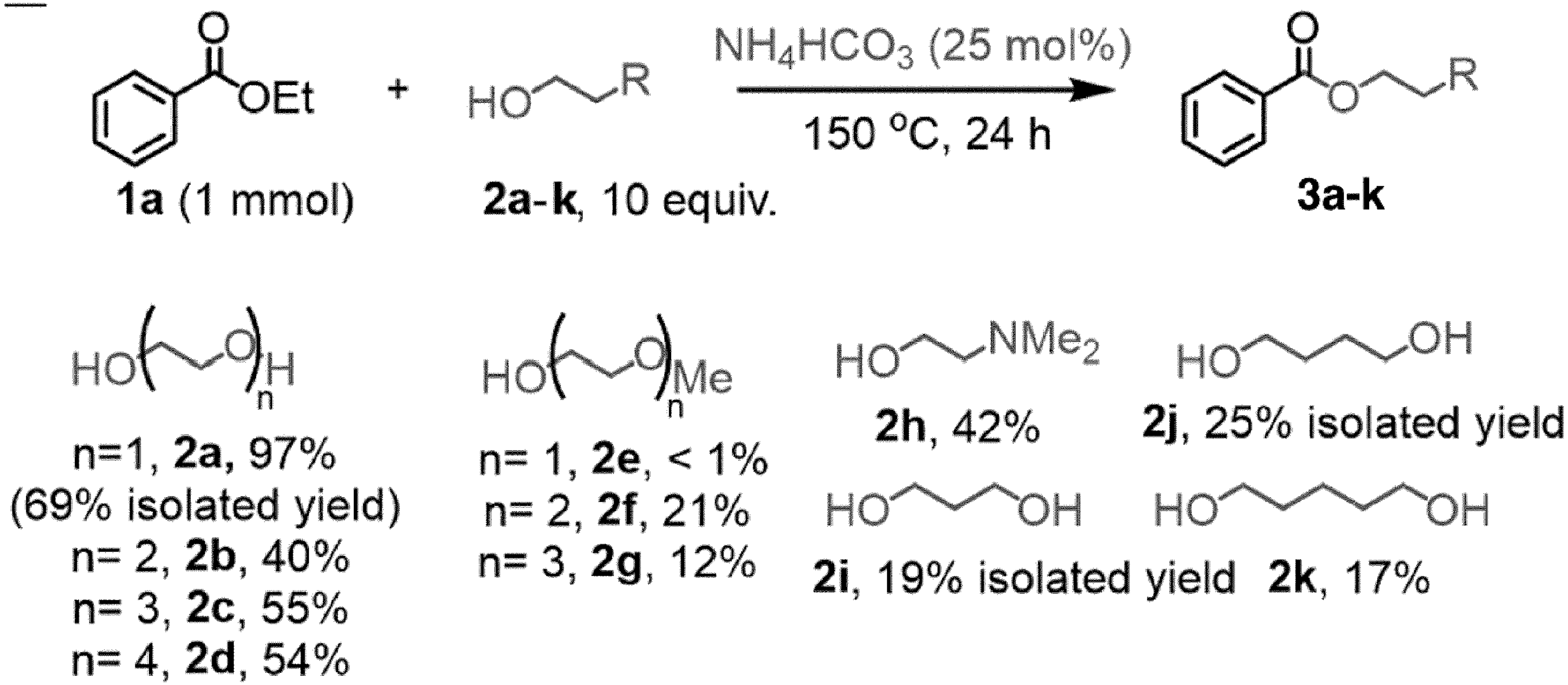
NH$_4$HCO$_3$ (25 mol%)
150 °C, 24 h
**1a** (1 mmol)
**2a-k**, 10 equiv.
**3a-k**
n=1, **2a,** 97%
(69% isolated yield)
n= 2, **2b**, 40%
n= 3, **2c**, 55%
n= 4, **2d**, 54%
n= 1, **2e**, < 1%
n= 2, **2f**, 21%
n= 3, **2g**, 12%
**2h**, 42%
**2j**, 25% isolated yield
**2i**, 19% isolated yield
**2k**, 17%

Fig. 2B

A

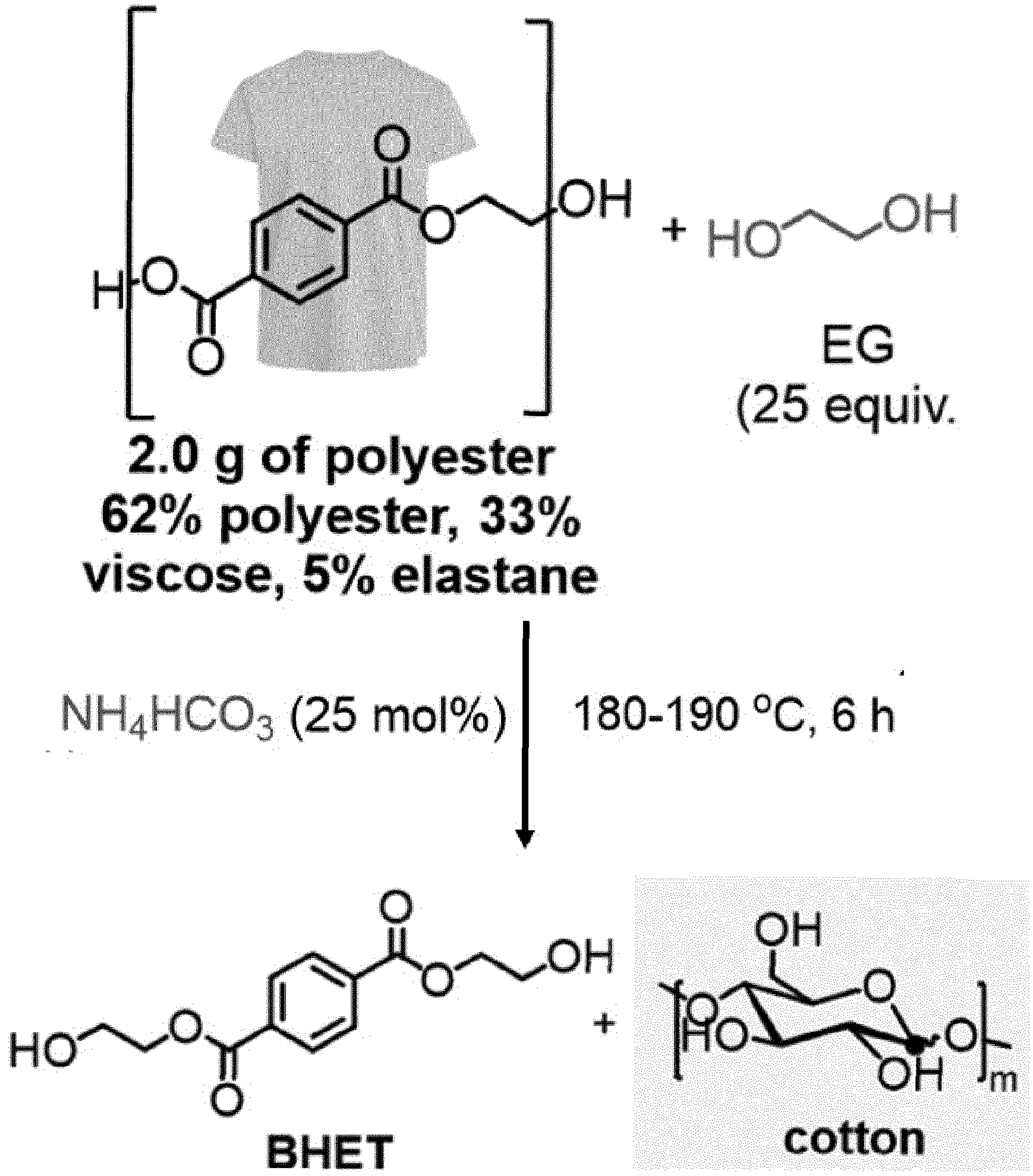
EG
(25 equiv.
2.0 g of polyester
62% polyester, 33%
viscose, 5% elastane
$NH_4HCO_3$ (25 mol%)
180-190 °C, 6 h
BHET
cotton

Fig. 3A

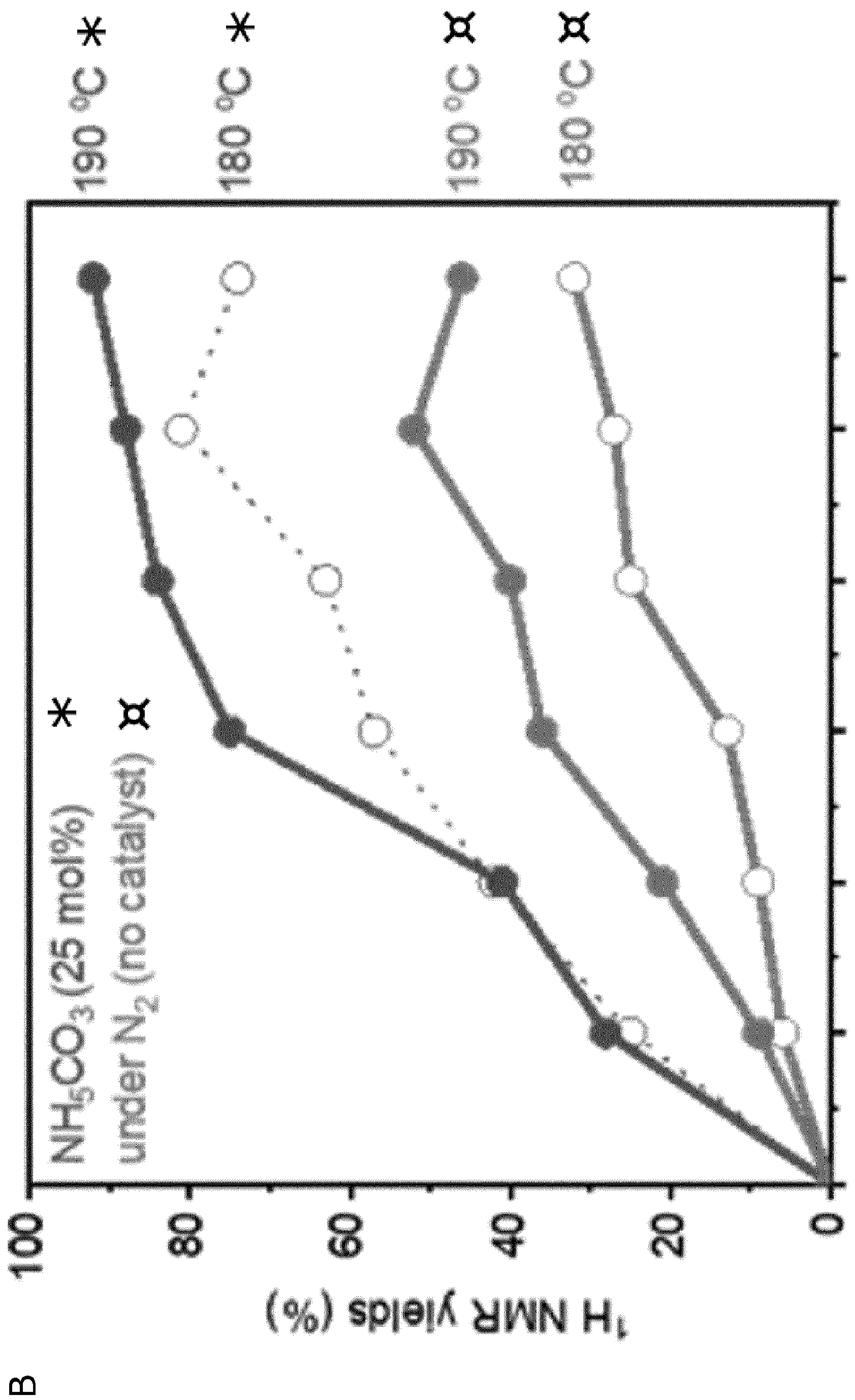
B
$NH_5CO_3$ (25 mol%)
under $N_2$ (no catalyst)
190 °C
180 °C
190 °C
180 °C
$^1H$ NMR yields (%)
0
20
40
60
80
100
0
1
2
3
4
5
6

Fig. 3B

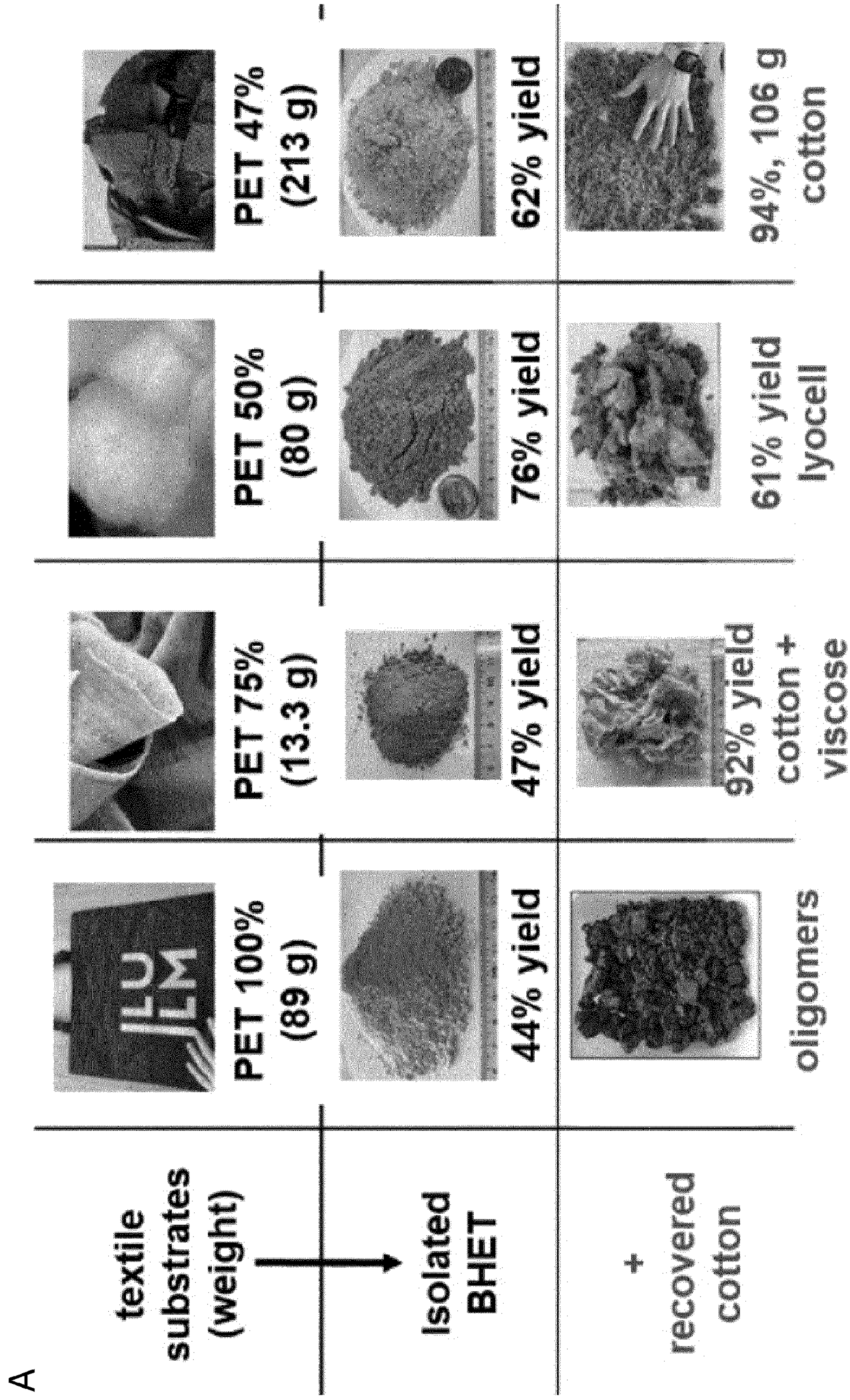
A
textile substrates (weight)
Isolated BHET
+ recovered cotton
PET 100% (89 g)
44% yield
oligomers
PET 75% (13.3 g)
47% yield
92% yield cotton + viscose
PET 50% (80 g)
76% yield
61% yield lyocell
PET 47% (213 g)
62% yield
94%, 106 g cotton

Fig. 4A

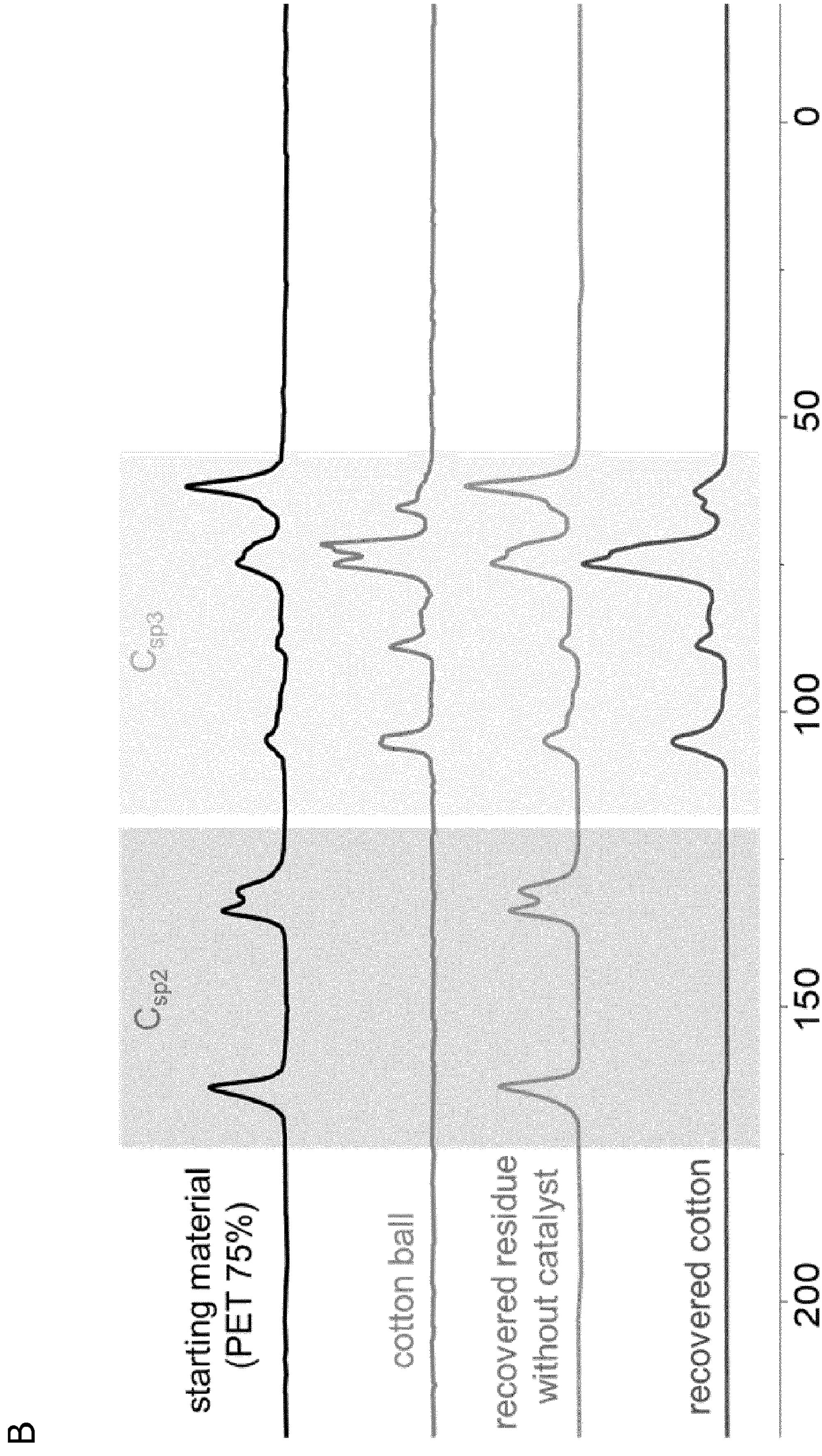
B
starting material
(PET 75%)
cotton ball
recovered residue
without catalyst
recovered cotton
C$_{sp2}$
C$_{sp3}$
200
150
100
50
0

Fig. 4B

C

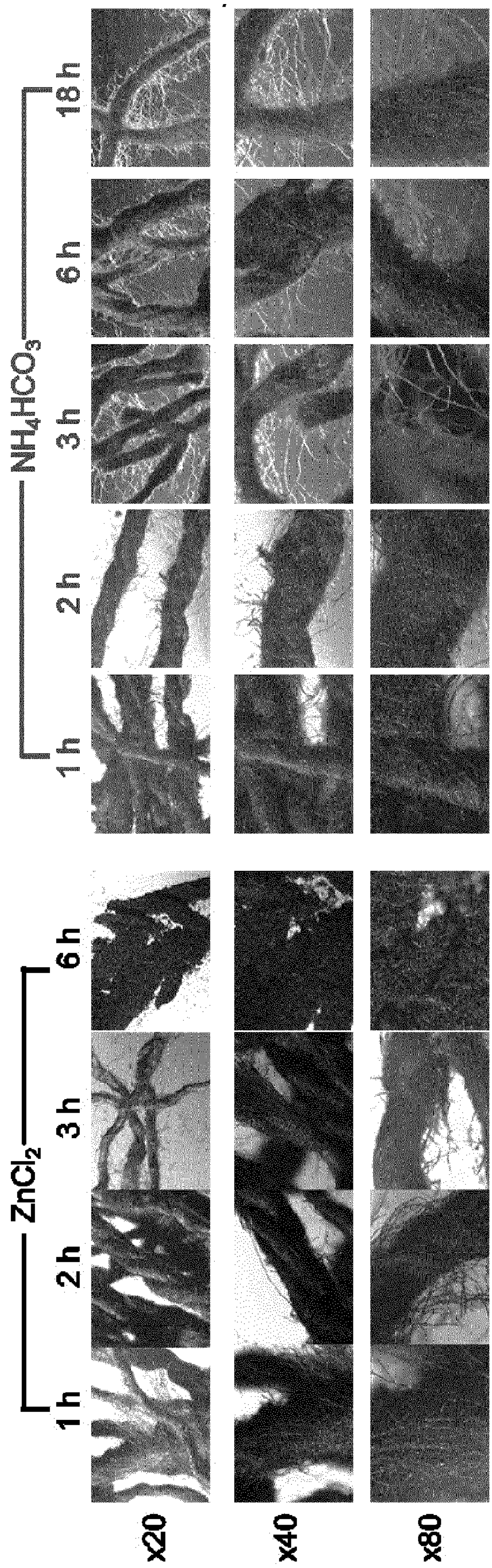


Fig. 4C

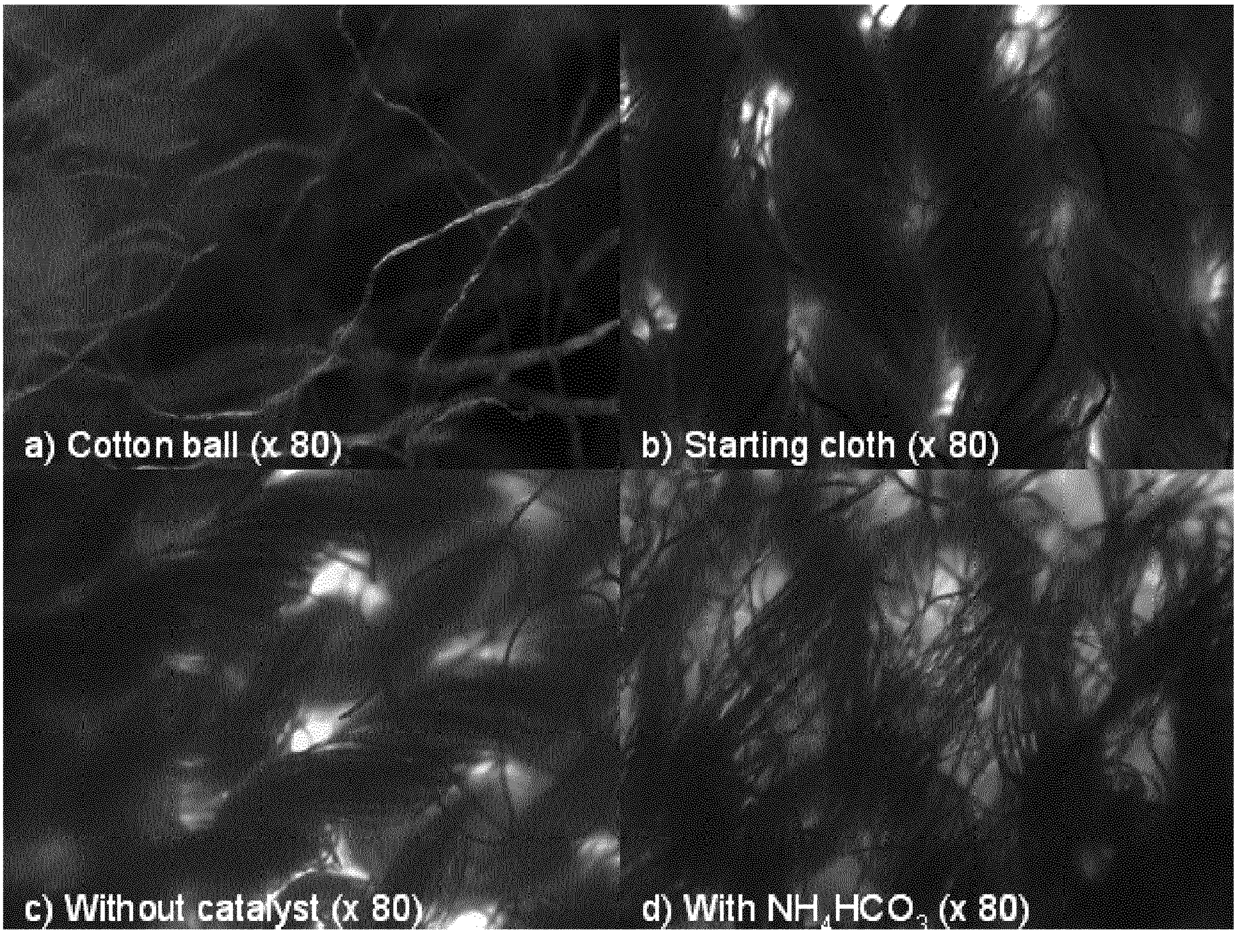
a) Cotton ball (x 80)
b) Starting cloth (x 80)
c) Without catalyst (x 80)
d) With NH4HCO3 (x 80)

Fig. 4D

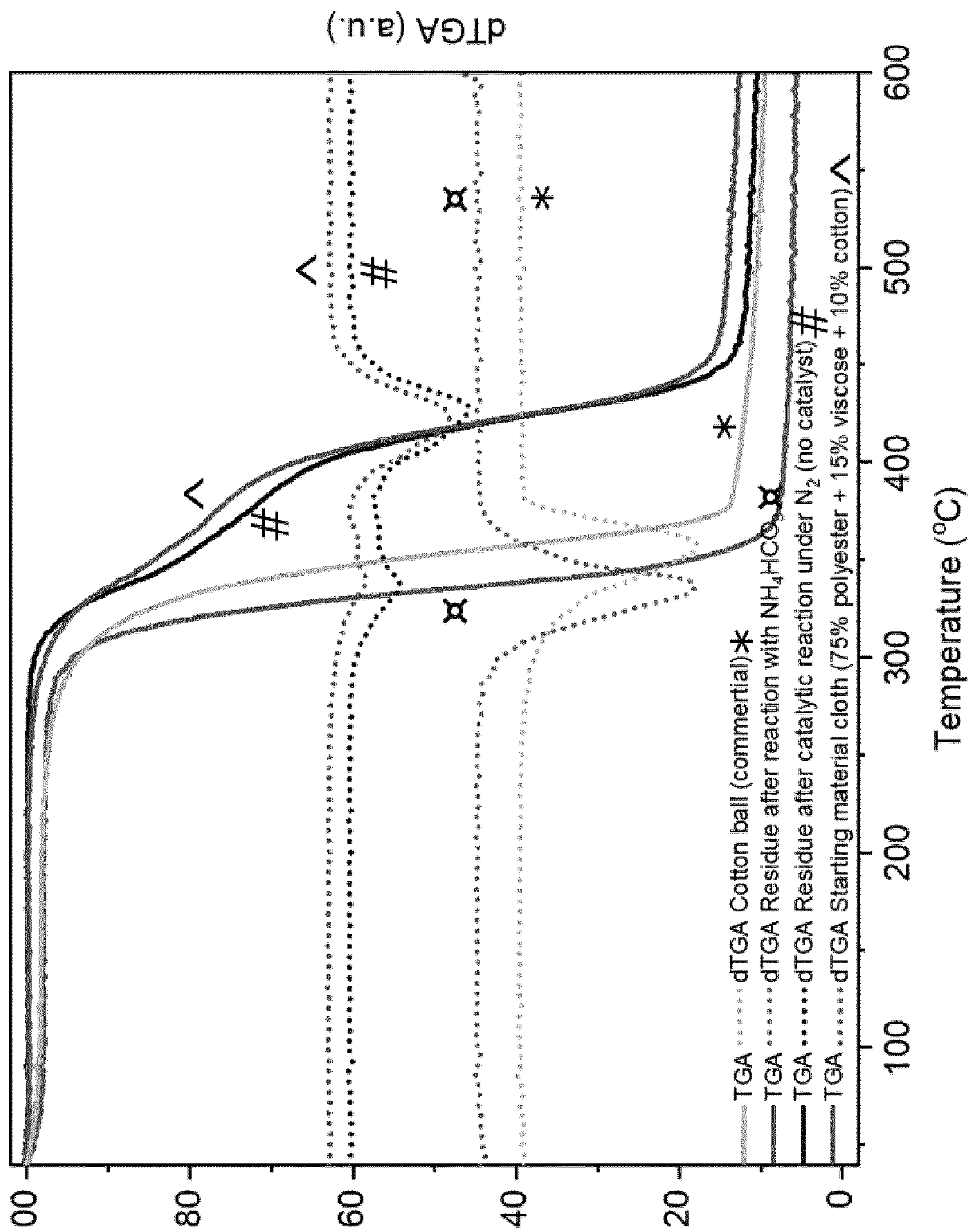
TGA dTGA Cotton ball (commercial) *
TGA dTGA Residue after reaction with $NH_4HCO_3$ ¤
TGA dTGA Residue after catalytic reaction under $N_2$ (no catalyst) #
TGA dTGA Starting material cloth (75% polyester + 15% viscose + 10% cotton) ^
Mass loss (%)
dTGA (a.u.)
Temperature (ºC)
0
20
40
60
80
100
100
200
300
400
500
600

A

Fig. 5A

B

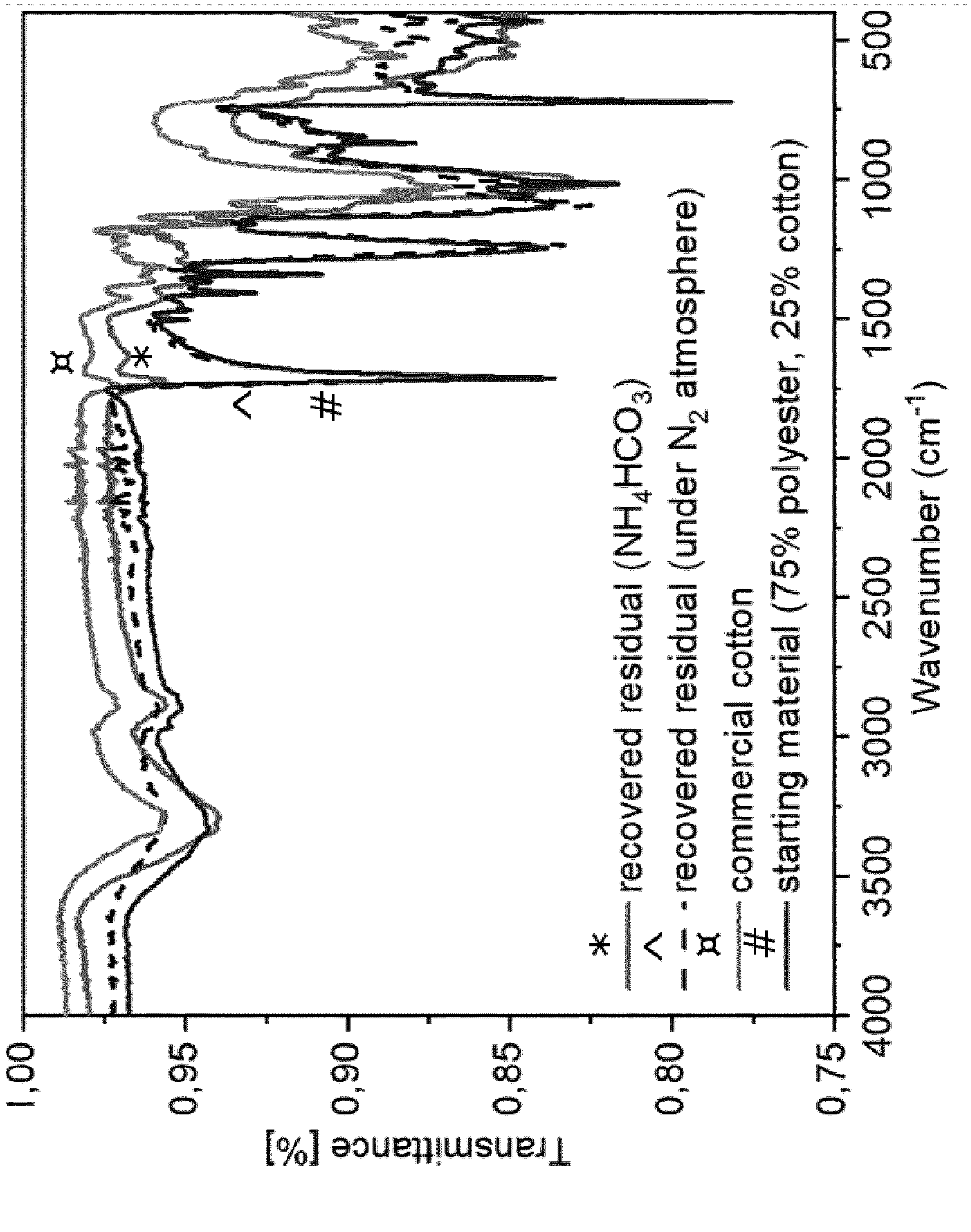

Transmittance [%]
1,00
0,95
0,90
0,85
0,80
0,75
4000
3500
3000
2500
2000
1500
1000
500
Wavenumber ($cm^{-1}$)
* recovered residual ($NH_4HCO_3$)
^ recovered residual (under $N_2$ atmosphere)
¤ commercial cotton
# starting material (75% polyester, 25% cotton)


Fig. 5B

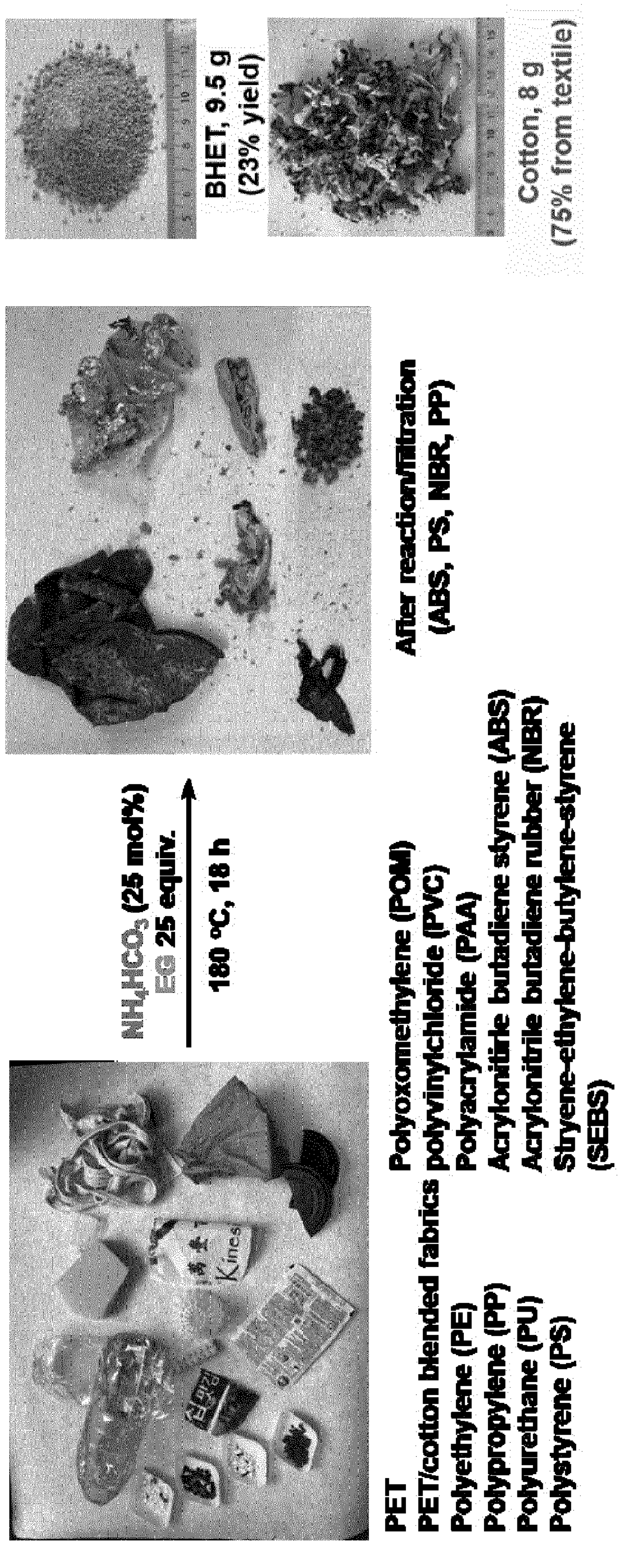
PET
PET/cotton blended fabrics
Polyethylene (PE)
Polypropylene (PP)
Polyurethane (PU)
Polystyrene (PS)
Polyoxomethylene (POM)
polyvinylchloride (PVC)
Polyacrylamide (PAA)
Acrylonitrile butadiene styrene (ABS)
Acrylonitrile butadiene rubber (NBR)
Stryene-ethylene-butylene-styrene (SEBS)
$NH_4HCO_3$ (25 mol%)
EG 25 equiv.
180 °C, 18 h
After reaction/filtration
(ABS, PS, NBR, PP)
BHET, 9.5 g
(23% yield)
Cotton, 8 g
(75% from textile)

Fig. 6

Europäisches Patentamt
European Patent Office
Office européen des brevets

# EUROPEAN SEARCH REPORT

Application Number
EP 23 15 0108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KR 101 888 612 B1 (SIONTECH CO LTD [KR] ET AL.) 14 August 2018 (2018-08-14)<br>* claim 1 *<br>----- | 1-15 | INV.<br>C07C69/82<br>C08J11/14<br>C08J11/24<br>C08J11/28 |
| X | YUE Q F ET AL: "Glycolysis of poly(ethylene terephthalate) (PET) using basic ionic liquids as catalysts", POLYMER DEGRADATION AND STABILITY, BARKING, GB, vol. 96, no. 4, 29 December 2010 (2010-12-29), pages 399-403, XP028365378, ISSN: 0141-3910, DOI: 10.1016/J.POLYMDEGRADSTAB.2010.12.020 [retrieved on 2011-01-20] | 1-11 | |
| Y | * table 1 *<br>----- | 12-15 | |
| X | US 2022/169824 A1 (BREYTA GREGORY [US] ET AL) 2 June 2022 (2022-06-02) | 12-15 | |
| Y | * example 4 *<br>----- | 12-15 | |
| | | | TECHNICAL FIELDS SEARCHED (IPC) |
| | | | C07C<br>C09J<br>C08J |

The present search report has been drawn up for all claims

1

| Place of search | Date of completion of the search | Examiner |
|---|---|---|
| The Hague | 2 May 2023 | Jansen, Reinier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 15 0108

This annex lists the patent family members relating to the patent documents cited in the above-mentioned European search report. The members are as contained in the European Patent Office EDP file on
The European Patent Office is in no way liable for these particulars which are merely given for the purpose of information.

02-05-2023

| Patent document cited in search report | | Publication date | Patent family member(s) | | Publication date |
|---|---|---|---|---|---|
| KR 101888612 | B1 | 14-08-2018 | NONE | | |
| US 2022169824 | A1 | 02-06-2022 | TW 202235509 | A | 16-09-2022 |
| | | | US 2022169824 | A1 | 02-06-2022 |
| | | | WO 2022118148 | A1 | 09-06-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015056377 A


### Non-patent literature cited in the description


- **M.GHAEMY et al.** *Polymer Degradation and Stability,* December 2005, vol. 90 (3), 570-576